# EUROPÄISCHE PATENTSCHRIFT

(11) **EP 2 200 958 B1**
(45) Veröffentlichungstag und Bekanntmachung des Hinweises auf die Patenterteilung: **04.11.2015**
(21) Anmeldenummer: 08803383.2
(22) Anmeldetag: 29.08.2008
(51) Int. Cl.: C07C 29/60, C07C 31/20, B01J 23/72

(54) **VERFAHREN ZUR HERSTELLUNG VON 1,2-PROPANDIOL DURCH HYDRIERUNG VON GLYCERIN IN EINER ZWEISTUFIGEN REAKTORKASKADE**
METHOD FOR PRODUCING 1,2-PROPANDIOL BY HYDROGENATING GLYCERINE IN A TWO-STEP REACTOR CASCADE
PROCÉDÉ DE PRODUCTION DE PROPANE-1,2-DIOL PAR HYDROGÉNATION DE GLYCÉRINE DANS UNE CASCADE DE RÉACTEURS À DEUX ÉTAGES

(30) Priorität: 31.08.2007 EP 07115460
(43) Veröffentlichungstag der Anmeldung: 30.06.2010
(73) Patentinhaber: BASF SE, 67056 Ludwigshafen (DE)
(72) Erfinder: HENKELMANN, Jochem, 68165 Mannheim (DE); PROCHAZKA, Roman, 68163 Mannheim (DE); BEY, Oliver, 67150 Niederkirchen (DE); MAURER, Stephan, 67435 Neustadt-Gimmeldingen (DE); STEINER, Jochen, 68165 Mannheim (DE); URTEL, Heiko, 68165 Mannheim (DE); THEIS, Gerhard, 67133 Maxdorf (DE); WAHL, Peter, 69118 Heidelberg (DE); MAIER, Petra, 68239 Mannheim (DE); MEHRL, Georg, 67117 Limburgerhof (DE)
(86) Internationale Anmeldenummer: PCT/EP2008/061385
(87) Internationale Veröffentlichungsnummer: WO 2009/027500

(56) Entgegenhaltungen:
- WO-A-2007/010299
- WO-A-2007/053705
- WO-A-2008/012244
- WO-A-2008/151784
- DE-A1- 4 302 464
- US-A- 5 616 817

## Beschreibung

Die vorliegende Erfindung betrifft ein Verfahren zur Herstellung von 1,2-Propandiol, bei dem man einen glycerinhaltigen Strom, insbesondere einen großtechnisch bei der Herstellung von Biodiesel anfallenden Strom, einer Hydrierung in einer zweistufigen Reaktorkaskade unterzieht.

Schwindende Erdölvorräte und steigende Kraftstoffpreise führen zu einem wachsenden Interesse, auf Erdölbasis hergestellte Kraftstoffe durch preiswerte und umweltschonende Alternativen zu ersetzen. Verfahren zur Herstellung von Kraftstoffen aus biogenen fett- oder ölhaltigen Ausgangsgemischen sowie beispielsweise in Restaurants anfallenden gebrauchten Ölen und tierischen Fetten sind seit längerem bekannt, wobei in Mitteleuropa derzeit überwiegend Rapsöl als Ausgangsprodukt bei der Produktion biogener Kraftstoffe eingesetzt wird. Biogene Öle und Fette selbst sind als Motorenkraftstoff weniger geeignet, da sie zuvor durch meist aufwändige Verfahren gereinigt werden müssen. Dazu zählt die Entfernung von Lecithinen, Kohlenhydraten und Proteinen, die Entfernung des so genannten Ölschlamms sowie die Entfernung der beispielsweise im Rapsöl in größeren Mengen enthaltenen freien Fettsäuren. So aufbereitete Pflanzenöle weichen dennoch von den technischen Eigenschaften herkömmlicher Dieselkraftstoffe in mehreren Punkten ab. So weisen sie in der Regel eine höhere Dichte als Dieselkraftstoff auf, die Cetanzahl von Rapsöl ist geringer als die von Dieselkraftstoff, und die Viskosität ist gegenüber der von Dieselkraftstoff um ein Vielfaches höher. Dies führt zu einer nicht akzeptablen Verschlechterung der Kraftstoffeigenschaften, wie zu einem ungleichmäßigen Laufverhalten des Motors, einer deutlich erhöhten Geräuschemission sowie durch die höhere Viskosität zu einer schlechteren Zerstäubung und Verbrennung im Brennraum. Die Verwendung von reinen Pflanzenölen führt daher in herkömmlichen Motoren zu Verkokungen, verbunden mit erhöhter Partikelemission. Zur Lösung dieser Probleme ist es bekannt, die in den biogenen Öl- und Fettausgangsgemischen enthaltenen Triglyceride (Fettsäureester des Glycerins) in Fettsäuremonoalkylester, insbesondere Methyl- oder Ethylester, umzuwandeln. Diese auch als "Biodiesel" bezeichneten Ester lassen sich in der Regel ohne größere Nachrüstungen in Dieselmotoren einsetzen, wobei der Ausstoß von unverbrannten Kohlenwasserstoffen und Rußpartikeln im Vergleich zu normalem Dieselkraftstoff vielfach sogar reduziert werden kann. Bei der Umesterung der Triglyceride zur Biodieselherstellung fällt auch Glycerin an (≈ 10 %), welches sowohl aus Gründen der Wirtschaftlichkeit als auch der Nachhaltigkeit einer Verwertung zugeführt werden soll. Es besteht daher ein Bedarf an effektiven und wirtschaftlichen Verfahren, die auch eine Verwertung des bei der Biodieselherstellung anfallenden Glycerins ermöglichen. Diese Verfahren sollen sich insbesondere auch zur Verwertung weiterer großtechnisch zur Verfügung stehender Glycerinströme eignen.

Die US 2,360,844 beschreibt ein Verfahren zur Herstellung von Seifen, bei dem man ein rohes Glycerid mit C₁-C₄-Alkanolen umestert und dabei das freigesetzte Glycerin von den Monoalkylestern abtrennt. Die Verwertung des dabei erhaltenen Glycerins ist nicht beschrieben.

Die US 5,354,878 beschreibt ein Verfahren zur Herstellung von Niederalkylestern höherer Fettsäuren mit einem geringen Restglyceringehalt durch Umesterung von Fettsäuretriglyceriden und die Verwendung dieser Ester als Dieselkraftstoff.

Die DE 102 43 700 A1 beschreibt ein druckloses Verfahren zur Herstellung von Alkylestern höherer Fettsäuren, insbesondere Biodiesel, aus freie Fettsäuren enthaltenden Fettsäuretriglycerid-Ausgangsgemischen mit einer Kombination aus saurer Veresterung und basischer Umesterung. Das bei der Umesterung anfallende Glycerin wird zum Teil als Schleppmittel bei der Veresterung der freien Fettsäuren eingesetzt.

Es ist bekannt, höherwertige Alkohole durch katalytische Hydrierung in niedrigerwertige Alkohole zu überführen. So beschreibt die DE-PS-524 101 ein solches Verfahren, bei dem man u. a. Glycerin einer Gasphasenhydrierung in Gegenwart eines Hydrierungskatalysators mit Wasserstoff in erheblichem Überschuss unterzieht. Konkret werden zur Hydrierung von Glycerin mit Cr aktivierte Kupfer- oder Kobaltkatalysatoren eingesetzt.

Die DE-PS-541 362 beschreibt ein Verfahren zur Hydrierung von Polyoxyverbindungen, wie z. B. Glycerin, in Gegenwart von Katalysatoren bei erhöhten Temperaturen über 150 °C und unter erhöhtem Druck. Konkret wird die Hydrierung von Glycerin mit einem Nickelkatalysator bei einer Temperatur von 200 bis 240 °C und einem Wasserstoffdruck von 100 atm beschrieben.

R. Connor und H. Adkins beschreiben in J. Am. Chem. Soc. 54, 1932, S. 4678 - 4690 die Hydrogenolyse von sauerstoffhaltigen organischen Verbindungen, u. a. von 98%igem Glycerin, zu 1,2-Propandiol in Gegenwart eines Kupfer-Chrom-BariumOxid-Katalysators. C. Montassier et al. beschreiben in Bulletin de la Societe Chimique de France 1989, Nr. 2, S. 148 - 155 Untersuchungen des Reaktionsmechanismus der katalytischen Hydrierung von Polyolen in Gegenwart diverser metallischer Katalysatoren, wie z. B. von Glycerin in Gegenwart von Raney-Kupfer.

J. Chaminand et al. beschreiben in Green Chem. 6, 2004, S. 359 - 361 die Hydrierung wässriger Glycerinlösungen bei 180 °C und 80 bar Wasserstoffdruck in Gegenwart von geträgerten Metallkatalysatoren auf Basis von Cu, Pd und Rh.

Die DE 43 02 464 A1 beschreibt ein Verfahren zur Herstellung von 1,2-Propandiol durch Hydrierung von Glycerin in Gegenwart eines heterogenen Katalysators bei Drücken von 20 bis 300 bar, insbesondere bei 100 bis 250 bar und Temperaturen von 150 °C bis 320 °C, wobei man Glycerin in dampfförmiger oder flüssiger Form über ein Katalysatorbett leitet. Als Katalysatoren werden u. a. Cu-Chromit, Cu-Zinkoxid, Cu-Aluminiumoxid und Cu-Siliciumdioxid genannt. Der Einsatz von glycerinhaltigen Strömen aus der Biodieselherstellung sowie Maßnahmen zur Vorbehandlung solcher Ströme vor ihrem Einsatz zur Hydrierung sind in diesem Dokument nicht beschrieben.

Die EP 0 523 015 beschreibt ein Verfahren zur katalytischen Hydrierung von Glycerin zur Herstellung von 1,2-Propandiol und 1,2-Ethandiol in Gegenwart eines Cu/Zn-Katalysators bei einer Temperatur von wenigstens 200 °C. Bei diesem Verfahren wird das Glycerin als wässrige Lösung mit einem Glyceringehalt von 20 bis 60 Gew.-% eingesetzt, der maximale Glyceringehalt in den Ausführungsbeispielen liegt bei 40 Gew.-%.

WO 2005/095536 beschreibt ein Niederdruckverfahren zur Umwandlung von Glycerin in Propylenglycol, bei dem man einen glycerinhaltigen Strom mit einem Wassergehalt von höchstens 50 Gew.-% bei einer Temperatur im Bereich von 150 bis 250 °C und einem Druck im Bereich von 1 bis 25 bar einer katalytischen Hydrierung unterzieht.

M. A. Dasari et al. beschreiben in Appl. Catalysis A: General 281, 2005, Seiten 225 - 231 ein Verfahren zur Niederdruckhydrierung von Glycerin zu Propylenglycol bei einer Temperatur von 200 °C und einem Wasserstoffdruck von 200 psi (13,79 bar) in Gegenwart eines Nickel-, Palladium-, Platin-, Kupfer- oder Kupfer-Chromit-Katalysators. Es wurden unterschiedliche Reaktionsparameter getestet, wie u. a. der Wassergehalt des eingesetzten Glycerins. Dabei nahm mit abnehmendem Wassergehalt zwar der Umsatz zu, die höchste Selektivität wurde bei diesem Niederdruckverfahren jedoch bei einem Wassergehalt von 20 Gew.-% erzielt.

Die US 5,616,817 beschreibt ein Verfahren zur Herstellung von 1,2-Propandiol durch katalytische Hydrierung von Glycerin bei erhöhter Temperatur und erhöhtem Druck, bei dem man Glycerin mit einem Wassergehalt von höchstens 20 Gew.-% in Gegenwart eines Katalysators, der 40 bis 70 Gew.-% Kobalt, gegebenenfalls Mangan und/oder Molybdän und einen geringen Gehalt an Kupfer von 10 bis 20 Gew.-% enthält, umsetzt. Die Temperatur liegt dabei in einem Bereich von etwa 180 bis 270 °C und der Druck in einem Bereich von 100 bis 700 bar, vorzugsweise 200 bis 325 bar.

Die WO 2007/053705 A2 beschreibt ein Verfahren zur Umwandlung von Glycerin in beliebige Mischungen aus Acetol und Propylenglycol, bei dem man das Reaktionsgemisch in der Gasphase mit einem heterogenen Katalysator in Kontakt bringt. Bevorzugt enthält die Gasphase Wasserstoff mit einem Partialdruck von 0,01 bis 25 bar.

Die WO 2007010299 A1 beschreibt ein Verfahren zur Hydrierung von Glycerin, bei dem man ein Glycerin-haltiges Ausgangsmaterial mit Wasserstoff in der Gasphase in Gegenwart eines Katalysators bei einer Temperatur von 160 bis 260°C, einem Druck von etwa 10 bis 30 bar, einem Verhältnis von Wasserstoff zu Glycerin von 400:1 bis 600:1 und einer Verweilzeit von etwa 0,01 bis 2,5 Stunden umsetzt.

Die DE 43 02 464 A1 beschreibt die Herstellung von 1,2-Propandiol aus Glycerin, wobei man Glycerin in Anwesenheit eines heterogenen Katalysators hydriert, der ein elementares Metall, ein Metallsalz, ein Metalloxid und/oder eine andere Metallverbindung und/oder -legierung der I. und/oder VIII. Nebengruppe enthält, zu 1,2-Propandiol mit verdünntem oder unverdünntem Wasserstoff bei Drücken von 20 bis 300 bar, insbesondere bei 100 bis 250 bar, und Temperaturen von 150°C bis 320°C in kontinuierlicher Fahrweise.

Die unveröffentlichte PCT/EP2007/051983 beschreibt ein Verfahren zur Herstellung von 1,2-Propandiol, bei dem man einen glycerinhaltigen Strom einer Hydrierung in Gegenwart eines kupferhaltigen, heterogenen Katalysators bei einer Temperatur von 100 bis 320 °C und einem Druck von 100 bis 325 bar unterzieht.

Der vorliegenden Erfindung liegt die Aufgabe zu Grunde, ein verbessertes Verfahren zur Herstellung von 1,2-Propandiol zur Verfügung zu stellen. So sind die bekannten Verfahren noch verbesserungswürdig hinsichtlich einer möglichst vollständigen Hydrierung des Glycerins bei gleichzeitig guter Selektivität bezüglich des gewünschten 1,2-Propandiol. Zudem benötigen viele aus dem Stand der Technik bekannte Verfahren relativ große Katalysatormengen oder weisen eine zu geringe Raum-Zeit-Ausbeute auf. Das bereitgestellte Verfahren soll sich insbesondere auch zur Weiterverarbeitung großtechnisch anfallender Glycerinströme eignen, wie sie bei der Umesterung von Fettsäuretriglyceriden zur Herstellung von Alkylestern höherer Fettsäuren anfallen.

Gegenstand der Erfindung ist daher ein Verfahren zur Herstellung von 1,2-Propandiol, bei dem man
a) einen glycerinhaltigen Strom bereitstellt, und
b) den glycerinhaltigen Strom einer kontinuierlichen Hydrierung in zwei hintereinander geschalteten Hydrierreaktoren in Gegenwart eines kupferhaltigen, heterogenen Katalysators unterzieht, wobei der Umsatz im ersten Reaktor wenigstens 80 %, bezogen auf den Glyceringehalt, beträgt wobei die kontinuierliche Hydrierung in beiden Hydrierreaktoren bei einem Druck im Bereich von 30 bar bis 300 bar erfolgt.

Das in Schritt b) erhaltene Hydrierungsprodukt kann gegebenenfalls wenigstens einem Aufarbeitungsschritt unterzogen werden (Schritt c)).

Erfindungsgemäß erfolgt die Hydrierung kontinuierlich in zwei hintereinander (in Reihe) geschalteten Hydrierreaktoren. Jeder Reaktor kann dabei eine oder mehrere Reaktionszonen innerhalb des Reaktors aufweisen. Bei den Reaktoren kann es sich um gleiche oder verschiedene Reaktoren handeln. Diese können z. B. jeweils gleiche oder verschiedene Vermischungscharakteristiken aufweisen und/oder durch Einbauten ein- oder mehrfach unterteilt sein.

Geeignete druckfeste Reaktoren für die Hydrierung sind dem Fachmann bekannt. Dazu zählen die allgemein üblichen Reaktoren für Gas-Flüssig-Reaktionen, wie z. B. Rohrreaktoren, Rohrbündelreaktoren, Gasumlaufreaktoren, Blasensäulen, Schlaufenapparate, Rührkessel (die auch als Rührkesselkaskaden ausgestaltet sein können), Air-Lift-Reaktoren etc.

Das erfindungsgemäße Verfahren unter Verwendung von heterogenen Katalysatoren kann in Festbett- oder Suspensionsfahrweise durchgeführt werden. Die Festbettfahrweise kann dabei z. B. in Sumpf- oder in Rieselfahrweise durchgeführt werden. Dabei werden die Katalysatoren vorzugsweise als Formkörper eingesetzt, wie sie im Folgenden beschrieben sind, z. B. in Form von gepressten Zylindern, Tabletten, Pastillen, Wagenrädern, Ringen, Sternen oder Strangpresslingen, wie Vollsträngen, polylobären Strängen, Hohlsträngen, Wabenkörpern, etc.

Bei der Suspensionsfahrweise werden ebenfalls heterogene Katalysatoren eingesetzt. Die heterogenen Katalysatoren werden dabei zumeist in feinverteiltem Zustand eingesetzt und liegen im Reaktionsmedium feinteilig suspendiert vor.

Geeignete heterogene Katalysatoren und Verfahren zu ihrer Herstellung werden im Folgenden näher beschrieben.

Bei der Hydrierung an einem Festbett wird wenigstens ein Reaktor eingesetzt, in dessen Innenraum ein Festbett angeordnet ist, durch das das Reaktionsmedium strömt. Das Festbett kann dabei aus einer einzigen oder aus mehreren Schüttungen gebildet sein. Jede Schüttung kann dabei eine oder mehrere Zonen aufweisen, wobei wenigstens eine der Zonen ein als Hydrierkatalysator aktives Material enthält. Jede Zone kann dabei ein oder mehrere verschiedene katalytisch aktive Materialien aufweisen und/oder ein oder mehrere verschiedene inerte Materialien aufweisen. Verschiedene Zonen können jeweils gleiche oder verschiedene Zusammensetzungen aufweisen. Es ist auch möglich, mehrere katalytisch aktive Zonen vorzusehen, die beispielsweise durch inerte Schüttungen voneinander getrennt sind. Die einzelnen Zonen können auch unterschiedliche katalytische Aktivität aufweisen. Dazu können verschiedene katalytisch aktive Materialien eingesetzt werden und/oder wenigstens einer der Zonen ein inertes Material beigemischt werden. Das Reaktionsmedium, das durch das Festbett strömt, enthält erfindungsgemäß mindestens eine flüssige Phase. Das Reaktionsmedium kann auch zusätzlich eine gasförmige Phase enthalten.

Als Reaktoren bei der Hydrierung in Suspension kommen insbesondere Schlaufenapparate, wie Strahlschlaufen oder Propellerschlaufen, Rührkessel, die auch als Rührkesselkaskaden ausgestaltet sein können, Blasensäulen oder Air-Lift-Reaktoren zum Einsatz.

Vorzugsweise erfolgt die kontinuierliche Hydrierung in Schritt b) in zwei hintereinander (in Reihe) geschalteten Festbettreaktoren. Die Reaktoren werden vorzugsweise im Gleichstrom betrieben. Die Einspeisung der Zuführströme kann sowohl von oben als auch von unten erfolgen.

Bei der Hydrierung liegen das Glycerin und das resultierende 1,2-Propandiol vorzugsweise in flüssiger Phase vor.

Die Temperatur bei der Hydrierung in Schritt b) beträgt in beiden Reaktoren im Allgemeinen etwa 150 bis 250 °C, insbesondere 170 bis 230 °C.

Gewünschtenfalls kann im zweiten Reaktor eine andere Temperatur eingestellt werden als im ersten Reaktor. In einer speziellen Ausführung wird der zweite Reaktor mit einer höheren Temperatur betrieben als der erste Reaktor. Zusätzlich kann der erste und/oder zweite Reaktor zwei oder mehr Reaktionszonen mit unterschiedlicher Temperatur aufweisen. So kann beispielsweise in einer zweiten Reaktionszone eine andere, vorzugsweise eine höhere, Temperatur als in der ersten Reaktionszone bzw. in jeder nachfolgenden Reaktionszone eine höhere Temperatur als in einer vorhergehenden Reaktionszone eingestellt werden, z. B. um einen möglichst vollständigen Umsatz bei der Hydrierung zu erzielen.

Der Reaktionsdruck in Schritt b) beträgt vorzugsweise in beiden Reaktoren 60 bis 250 bar, insbesondere 140 bis 250 bar.

Gewünschtenfalls kann im zweiten Reaktor ein anderer Druck eingestellt werden als im ersten Reaktor. In einer speziellen Ausführung wird der zweite Reaktor mit einem höheren Druck betrieben als der erste Reaktor.

Die Einspeisung des für die Hydrierung benötigten Wasserstoffs kann in den ersten und gegebenenfalls zusätzlich in den zweiten Reaktor erfolgen. Vorzugsweise erfolgt die Einspeisung von Wasserstoff nur in den ersten Reaktor. Die den Reaktoren zugeführte Wasserstoffmenge ergibt sich aus der in der Hydrierreaktion verbrauchten Wasserstoffmenge und der gegebenenfalls mit dem Abgas ausgetragenen Wasserstoffmenge. Das Molverhältnis von Wasserstoff zu Glycerin beträgt vorzugsweise 1 : 1 bis 500 : 1, speziell 1,1 : 1 bis 100 : 1. Vorzugsweise wird der Wasserstoff in einem stöchiometrischen Überschuss von etwa 2 bis 25 Mol-%, besonders bevorzugt 5 bis 15 Mol-%, bezogen auf Glycerin eingesetzt.

Die Katalysator-Belastung bei kontinuierlicher Fahrweise beträgt vorzugsweise 0,05 bis 1, besonders bevorzugt 0,1 bis 0,5 kg, insbesondere 0,1 bis 0,3 kg zu hydrierendes Glycerin pro kg (Katalysator) pro h.

Der Umsatz im ersten Reaktor, bezogen auf das in dem glycerinhaltigen Strom enthaltene Glycerin, beträgt vorzugsweise wenigstens 85 %, besonders bevorzugt wenigstens 90 %, insbesondere wenigstens 92 %. Die Regelung des im ersten Reaktor umgesetzten Glycerinanteils kann z. B. über das Reaktorvolumen und/oder die Verweilzeit im ersten Reaktor erfolgen. Der Gesamtglycerinumsatz in Schritt b), bezogen auf das in dem glycerinhaltigen Strom enthaltene Glycerin, beträgt vorzugsweise wenigstens 97 %, besonders bevorzugt wenigstens 98 %, insbesondere wenigstens 99 %.

Zur Abfuhr der bei der exothermen Hydrierung entstehenden Reaktionswärme kann der erste und/oder der zweite Reaktor mit wenigstens einer Kühlvorrichtung versehen sein. Die Abfuhr der Reaktionswärme kann durch Kühlung eines externen Umlaufstroms oder durch interne Kühlung in wenigstens einem der Reaktoren erfolgen. Zur internen Kühlung können die dafür üblichen Vorrichtungen, im Allgemeinen Hohlkörpermodule, wie Field-Rohre, Rohrschlangen, Wärmetauscherplatten, etc. eingesetzt werden. Alternativ kann die Umsetzung auch in einem gekühlten Rohrbündelreaktor erfolgen.

Bevorzugt wird zur Hydrierung in Schritt b) eine zweistufige Reaktorkaskade eingesetzt, wobei der erste Hydrierreaktor über einen in einem externen Kreislauf geführten Strom aus der Reaktionszone verfügt (externer Umlaufstrom, Flüssigkeitsumlauf).

Sofern das im zweiten Reaktor hydrierte Reaktionsgemisch nur noch so geringe Anteile an hydrierbarem Glycerin aufweist, dass die bei der Reaktion auftretende Wärmetönung nicht ausreicht, die erwünschte Temperatur im Reaktor zu halten, kann auch eine Erwärmung des zweiten Reaktors (oder einzelner Reaktionszonen des zweiten Reaktors) erforderlich sein. Diese kann analog der zuvor beschriebenen Abfuhr der Reaktionswärme durch Erwärmung eines externen Umlaufstroms oder durch interne Erwärmung erfolgen. In einer geeigneten Ausführung kann zur Temperierung des zweiten Reaktors die Reaktionswärme aus dem ersten Reaktor verwendet werden.

Des Weiteren kann die dem Reaktionsgemisch entzogene Reaktionswärme zur Erwärmung der Edukte verwendet werden. Dazu kann z. B. der Glycerinzulaufstrom zumindest teilweise mit einem externen Umlaufstrom gemischt und die vereinigten Ströme dann in den ersten Reaktor geführt werden. Des Weiteren kann der Glycerinzulaufstrom mit Hilfe eines Wärmetauschers erwärmt werden, der mit einem der beiden Reaktoren entzogener Wärme betrieben wird.

In einer speziellen Ausgestaltung des Verfahrens wird eine Reaktorkaskade aus zwei in Reihe geschalteten Reaktoren eingesetzt, wobei die Umsetzung in dem zweiten Reaktor adiabatisch durchgeführt wird. Dieser Begriff wird im Rahmen der vorliegenden Erfindung im technischen und nicht im physiko-chemischen Sinne verstanden. So erfährt das Reaktionsgemisch beim Strömen durch den zweiten Reaktor auf Grund der exothermen Hydrierungsreaktion in der Regel eine Temperaturerhöhung. Unter adiabatischer Reaktionsführung wird eine Vorgehensweise verstanden, bei der die bei der Hydrierung freiwerdende Wärmemenge von der Reaktionsmischung im Reaktor aufgenommen und keine Kühlung durch Kühlvorrichtungen angewandt wird. Somit wird die Reaktionswärme mit dem Reaktionsgemisch aus dem zweiten Reaktor abgeführt, abgesehen von einem Restanteil, der durch natürliche Wärmeleitung und Wärmeabstrahlung vom Reaktor an die Umgebung abgegeben wird. Vorzugsweise wird der zweite Reaktor im geraden Durchgang betrieben.

In einer Ausführungsform kann in wenigstens einem der eingesetzten Reaktoren eine zusätzliche Durchmischung erfolgen. Eine zusätzliche Durchmischung ist insbesondere vorteilhaft, wenn die Hydrierung bei großen Verweilzeiten des Reaktionsgemischs erfolgt. Zur Durchmischung können z. B. die in die Reaktoren eingeführten Ströme eingesetzt werden, indem man diese über geeignete Mischvorrichtungen, wie Düsen, in die jeweiligen Reaktoren einführt. Zur Durchmischung können auch in einem externen Kreislauf geführte Ströme aus dem jeweiligen Reaktor eingesetzt werden.

Zur Vervollständigung der Hydrierung wird dem ersten Reaktor ein Austrag entnommen, der noch hydrierbares Glycerin enthält. In einer speziellen Ausführung wird dem ersten Reaktor ein Austrag entnommen, in einen ersten und einen zweiten Teilstrom aufgetrennt, wobei der erste Teilstrom als Kreisstrom dem ersten Reaktor wieder zugeführt wird und der zweite Teilstrom dem zweiten Reaktor zugeführt wird. Der Austrag kann gelöste oder gasförmige Anteile an Wasserstoff enthalten. In speziellen Ausführung wird der Austrag aus dem ersten Reaktor einem Phasentrennbehälter zugeführt, in eine flüssige und in eine gasförmige Phase getrennt, die flüssige Phase in den ersten und den zweiten Teilstrom getrennt und die Gasphase zumindest teilweise dem zweiten Reaktor separat zugeführt. In einer alternativen Ausführung wird der Austrag aus dem ersten Reaktor einem Phasentrennbehälter zugeführt und in einen ersten flüssigen an Wasserstoff abgereicherten Teilstrom und einen zweiten an Wasserstoff angereicherten Teilstrom auftrennt. Der erste Teilstrom wird dann als Kreisstrom dem ersten Reaktor wieder zugeführt und der zweite Teilstrom wird dem zweiten Reaktor (als glycerin- und wasserstoffhaltiger Feed) zugeführt. In einer weiteren alternativen Ausführung erfolgt die Beschickung des zweiten Reaktors mit Wasserstoff nicht über einen dem ersten Reaktor entnommenen wasserstoffhaltigen Feed, sondern mit frischem Wasserstoff über eine separate Zuleitung.

Bevorzugt ist eine Ausgestaltung des Verfahrensschritts b), bei der man
b1) in den ersten Reaktor eines Reaktionssystems, das aus einem Reaktor mit einen in einem externen Kreislauf geführten Strom (Kreislaufreaktor) und einem nachgeschalteten im geraden Durchgang betrieben Reaktor (adiabatischen Strömungsrohrreaktor) besteht, einen glycerinhaltigen Zulauf und Wasserstoff einspeist und in Gegenwart eines kupferhaltigen, heterogenen Katalysators bis zu einem Teilumsatz umsetzt,
b2) aus dem ersten Reaktor einen Austrag entnimmt, der Wasserstoff, Glycerin und 1,2-Propandiol enthält,
b3) den Austrag in einen ersten flüssigen an Wasserstoff abgereicherten Teilstrom und einen zweiten an Wasserstoff angereicherten Teilstrom auftrennt,
b4) den ersten Teilstrom nach Entzug eines Teils der enthaltenen Wärme in den ersten Reaktor zurückführt,
b5) den zweiten Teilstrom in den zweiten Reaktor einspeist und in Gegenwart eines kupferhaltigen, heterogenen Katalysators weiter umsetzt.

Die zuvor beschriebene Verfahrensvariante, eignet sich besonders vorteilhaft zur Steuerung der Reaktionstemperatur und des Wärmeübergangs zwischen Reaktionsmedium, begrenzenden Apparatewänden und Umgebung. Eine weitere Möglichkeit zur Steuerung der Wärmebilanz besteht in der Regelung der Eintrittstemperatur des glycerinhaltigen Zulaufs. So führt eine tiefere Temperatur des eintretenden Zulaufs in der Regel zu einer verbesserten Abführung der Hydrierwärme. Beim Nachlassen der Katalysatoraktivität kann die Eintrittstemperatur höher gewählt werden, um eine höhere Reaktionsgeschwindigkeit zu erreichen und somit die nachlassende Katalysatoraktivität zu kompensieren. Vorteilhafterweise kann so die Standzeit des eingesetzten Katalysators in der Regel verlängert werden.

Der erste Teilstrom wird im Allgemeinen chemisch unverändert in das Reaktionssystem zurückgeführt. Gewünschtenfalls können die Temperatur und/oder der Druck vor der Zurückführung auf die gewünschten Werte eingestellt werden. Die Einspeisung des ersten Teilstroms in den ersten Reaktor kann gemeinsam mit dem glycerinhaltigen Zulauf oder separat davon erfolgen. Das Gewichtsmengenverhältnis von in den ersten Reaktor eingespeistem ersten Teilstrom zu frischem glycerinhaltigen Zulauf liegt vorzugsweise in einem Bereich von 1 : 1 bis 50 : 1, besonders bevorzugt von 2 : 1 bis 30 : 1.

In einer speziellen Ausgestaltung wird auch dem zweiten Teilstrom vor Eintritt in den zweiten Reaktor Wärme entzogen. Dazu kann ein üblicher Wärmetauscher eingesetzt werden, der es ermöglicht, die gewonnene Wärmemenge an anderer Stelle des Verfahrens, z. B. bei der Auftrennung des Reaktionsaustrags aus dem zweiten Reaktor, wieder einzusetzen.

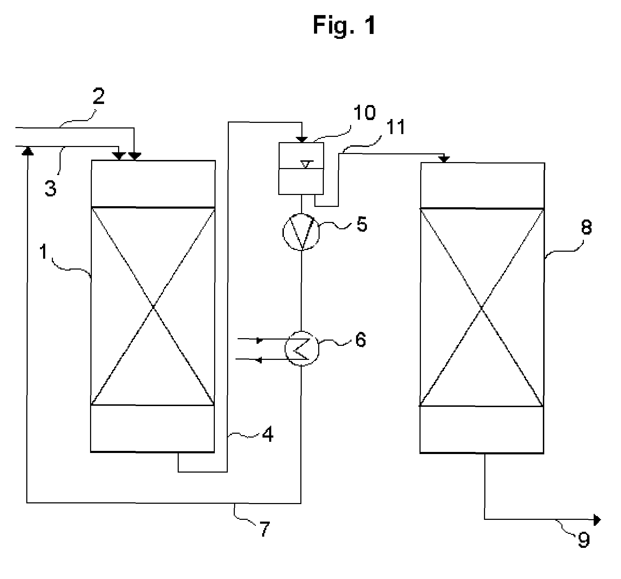

Figur 1 zeigt die schematische Darstellung einer zur Durchführung des Hydrierverfahrens geeigneten zweistufigen Reaktorkaskade, wobei aus Gründen der Übersichtlichkeit auf die Wiedergabe solcher Details verzichtet ist, die für die Erläuterung der Erfindung nicht relevant sind. Die Anlage umfasst einen ersten Hydrierreaktor (1) und einen zweiten Hydrierreaktor (8). Der Hydrierreaktor (1) ist als Kreislaufreaktor und der Hydrierreaktor (8) als adiabatischer Strömungsrohrreaktor ausgeführt. Über die Rohrleitung (2) wird Wasserstoffgas unter Druck in den Reaktor (1) eingeleitet. Über die Rohrleitung (3) wird ein glycerinhaltiger Strom in den Reaktor (1) eingeleitet. Über die Rohrleitung (4) und die Pumpe (5) wird ein Austrag aus dem Reaktor (1) entnommen und dem Phasentrennbehälter (10) zugeführt. Die im Phasentrennbehälter (10) erhaltene flüssige Phase wird im Wärmetauscher (6) abgekühlt und als Kreisstrom (7) dem Reaktor (1) wieder zugeführt. Der Phasentrennbehälter (10) kann, wie dargestellt, separat ausgeführt oder alternativ in den Reaktor (1) integriert sein. In dem Phasentrennbehälter (10) wird ein weiterer Strom (11) abgetrennt, der gasförmige und flüssige Anteile enthält und über die separate Leitung (11) dem Reaktor (8) zugeführt. In einer weiteren alternativen Ausführung erfolgt die Beschickung des Reaktors (8) mit Wasserstoff nicht über einen dem Reaktor (1) entnommenen Feed, sondern mit frischem Wasserstoff über eine separate Zuleitung. Das Hydrierprodukt verlässt Reaktor (8) über die Rohrleitung (9).

Für den Einsatz in dem erfindungsgemäßen Verfahren eignen sich prinzipiell alle glycerinhaltigen Ströme, auch solche aus technisch ausgeübten Verfahren und mit den dabei anfallenden Reinheitsgraden. Dazu zählen insbesondere glycerinhaltige Ströme aus der Verarbeitung öl- und/oder fetthaltiger Ausgangsstoffe, z. B. aus der Seifenherstellung, der Fettsäure- und Fettsäureesterherstellung etc. Vorzugsweise handelt es sich bei dem in Schritt a) bereitgestellten glycerinhaltigen Strom um einen bei der Herstellung von Alkylestern höherer Fettsäuren durch Umesterung von Fettsäuretriglyceriden anfallenden glycerinhaltigen Strom, wie er insbesondere bei der Herstellung von "Biodiesel" anfällt. Diese Ausführungsform des erfindungsgemäßen Verfahrens wird im Folgenden noch näher beschrieben.

Der in Schritt a) eingesetzte glycerinhaltige Strom weist vorzugsweise einen Wassergehalt von höchstens 30 Gew.-%, bevorzugt von höchstens 20 Gew.-%, auf. Besonders bevorzugt ist ein Wassergehalt entsprechend dem Glycerin-Monohydrat (Wassergehalt 16,3 Gew.-%) oder weniger. In einer speziellen Ausführungsform wird ein glycerinhaltiger Strom eingesetzt, der im Wesentlichen wasserfrei ist. Unter "im Wesentlichen wasserfrei" wird im Rahmen der vorliegenden Erfindung ein Wassergehalt von höchstens 3 Gew.-%, besonders bevorzugt von höchstens 1 Gew.-%, verstanden. Der Einsatz von glycerinhaltigen Strömen mit einem Wassergehalt im Bereich bis 30 Gew.-%, insbesondere bis 20 Gew.-%, ermöglicht in dem zur Hydrierung eingesetzten Temperatur- und Druckbereich die Herstellung von 1,2-Propandiol mit hohen Ausbeuten und mit hoher Selektivität. Die Hydrierung von glycerinhaltigen Strömen, die nicht im Wesentlichen wasserfrei sind und insbesondere von Strömen, die einen höheren Wassergehalt aufweisen als das Glycerinmonohydrat, ist ebenfalls mit hohen Ausbeuten und Selektivitäten möglich, allerdings auf Grund der verringerten Raum-Zeit-Ausbeute weniger wirtschaftlich. Dennoch kann ein Wassergehalt im Bereich von 3 bis 30 Gew.-% vorteilhaft für die rheologischen Eigenschaften während der Hydrierung sein. Eine spezielle Ausführungsform des erfindungsgemäßen Verfahrens betrifft daher den Einsatz glycerinhaltiger Ströme mit einem Wassergehalt im Bereich von 3 bis 30 Gew.-%, bevorzugt von 5 bis 20 Gew.-%, zur Verringerung der Viskosität bei der Hydrierung.

Die glycerinhaltigen Ströme können anstelle von oder zusätzlich zu Wasser wenigstens ein weiteres, vorzugsweise glycerinmischbares (und damit in der Regel auch wassermischbares), organisches Lösungsmittel aufweisen. Bevorzugt weisen die in Schritt a) bereitgestellten glycerinhaltigen Ströme einen Gesamtlösungsmittelgehalt von höchstens 20 Gew.-%, besonders bevorzugt höchstens 15 Gew.-%, insbesondere höchstens 10 Gew.-% und speziell höchstens 5 Gew.-% auf. Werden Lösungsmittelgemische eingesetzt, die Wasser und wenigstens ein glycerin- bzw. wassermischbares organisches Lösungsmittel enthalten, so beträgt der Anteil des organischen Lösungsmittels vorzugsweise höchstens 50 Gew.-%, besonders bevorzugt höchstens 20 Gew.-%, bezogen auf das Gesamtgewicht des Lösungsmittels. Geeignete glycerinmischbare organische Lösungsmittel sind C₁-C₄-Alkanole, wie Methanol, Ethanol, n-Propanol, Isopropanol, n-Butanol, tert.-Butanol, Polyole und Mono- und Dialkylether davon, cyclische Ether, wie Dioxan und Tetrahydrofuran, etc. Geeignete Lösungsmittel sind auch aromatische Kohlenwasserstoffe, wie Benzol, Toluol oder die Xylole. Bevorzugt als organische Lösungsmittel sind C₁-C₄-Alkanole, insbesondere Methanol und/oder Ethanol und deren Gemische mit Wasser. Bevorzugt weisen die in Schritt a) eingesetzten glycerinhaltigen Ströme jedoch keine organischen Lösungsmittel auf.

Die in Schritt a) bereitgestellten glycerinhaltigen Ströme können wenigstens einem Aufarbeitungsschritt unterzogen werden. Dazu zählt z. B. wenigstens ein Reinigungsschritt zur Entfernung unerwünschter Komponenten. Dazu zählt weiterhin eine Verringerung des Gehalts an Wasser und/oder, soweit vorhanden, organischer Lösungsmittel.

Je nach Herkunft können die glycerinhaltigen Ströme noch anorganische Salze als unerwünschte Komponente enthalten. Diese können aus dem Rohglycerin nach den im Folgenden beschriebenen Aufarbeitungsverfahren entfernt werden. Dazu eignet sich insbesondere eine thermische Aufarbeitung (z. B. unter Einsatz eines Sambay-Verdampfers).

Je nach Herkunft können die glycerinhaltigen Ströme auch Katalysatorgifte, d. h. Komponenten, welche die Hydrierung beeinträchtigen, indem sie den Hydrierungskatalysator deaktivieren, enthalten. Hierzu zählen z. B. stickstoffhaltige Verbindungen, wie Amine, und schwefelhaltige Verbindungen, wie Schwefelsäure, Schwefelwasserstoff, Thioalkohole, Thioether, z. B. Dimethylsulfid und Dimethyldisulfid, Kohlenoxidsulfid, Aminosäuren, z. B. Schwefel- und zusätzliche Stickstoffgruppen enthaltende Aminosäuren, Fettsäuren und deren Salz, etc. Zu den Katalysatorgiften zählen weiterhin Halogenverbindungen, Spuren von üblichen Extraktionsmitteln, z. B. Acetonitril oder N-Methylpyrrolidon, etc. sowie gegebenenfalls organische Phosphor- und Arsenverbindungen. Ein in glycerinhaltigen Strömen aus der Öl- und Fettveredelung häufig enthaltenes Katalysatorgift ist Schwefelsäure, welches als Katalysator bei der Veresterung bzw. Umesterung eingesetzt wird.

Zur Aufarbeitung der glycerinhaltigen Ströme in Schritt a) kann z. B. eine thermische Aufarbeitung, bevorzugt eine Destillation, eine Adsorption, ein Ionenaustausch, ein Membrantrennverfahren, eine Kristallisation, eine Extraktion oder eine Kombination aus zwei oder mehreren dieser Verfahren zum Einsatz kommen. Membrantrennverfahren unter Einsatz von Membranen definierter Porengrößen eignen sich speziell zur Verringerung des Wassergehalts und/oder zur Salzentfernung. Unter Kristallisation wird auch das partielle Ausfrieren der glycerinhaltigen Ströme an gekühlten Oberflächen verstanden. Somit lassen sich Verunreinigungen entfernen, die sich in der festen Phase anreichern.

In einer ersten Ausführung wird der glycerinhaltige Strom in Schritt a) einer Destillation zur Verringerung des Wassergehalts und/oder zur Entfernung von Komponenten, welche die katalytische Hydrierung beeinträchtigen, unterzogen. Diese kann prinzipiell nach üblichen, dem Fachmann bekannten Destillationsverfahren erfolgen. Geeignete Vorrichtungen zur destillativen Aufarbeitung umfassen Destillationskolonnen, wie Bodenkolonnen, die mit Glocken, Siebplatten, Siebböden, Packungen, Füllkörpern, Ventilen, Seitenabzügen, etc. ausgerüstet sein können, Verdampfer, wie Dünnschichtverdampfer, Fallfilmverdampfer, Zwangsumlaufverdampfer, Sambay-Verdampfer, etc. und Kombinationen davon. Die Entfernung von Schwefelsäure gelingt bereits durch eine einfache Destillation, insbesondere eine Kurzwegdestillation.

Geeignete Trennverfahren werden in den folgenden Dokumenten beschrieben: Sattler, Klaus: Thermische Trennverfahren, 3. Auflage, Wiley VCH, 2001; Schlünder E. U., Thurner F.: Destillation, Absorption, Extraktion, Springer Verlag, 1995; Mersmann, Alfons: Thermische Verfahrenstechnik, Springer Verlag, 1980; Grassmann P., Widmer F.: Einführung in die thermische Verfahrenstechnik, de Gruyter, 1997; Weiß S., Militzer K.-E., Gramlich K.: Thermische Verfahrenstechnik, Dt. Verlag für Grundstoffindustrie, Leipzig, Stuttgart, 1993. Auf diese Dokumente wird hier Bezug genommen.

In einer weiteren Ausführung wird der glycerinhaltige Strom in Schritt a) zur Verringerung des Gehalts an schwefelhaltigen Verbindungen, speziell schwefelhaltigen aromatischen Verbindungen, einer katalytischen Entschwefelung, gegebenenfalls in Gegenwart von Wasserstoff, unterzogen. Geeignete Entschwefelungsmittel umfassen eine Metallkomponente, wobei das Metall vorzugsweise ausgewählt ist unter Metallen der Gruppen 6, 7, 8, 9, 10, 11 und 12 des Periodensystems. Vorzugsweise werden die Metalle ausgewählt unter Mo, Ni, Cu, Ag, Zn und Kombinationen davon. Geeignete weitere Komponenten der Entschwefelungsmittel sind Dotierstoffe. Die Metallkomponente kann in oxidischer Form, reduzierter Form oder einer Mischung, die oxidierte und reduzierte Komponenten enthält, eingesetzt werden. Die aktive Komponente der Entschwefelungsmittel (Metallkomponente(n) und gegebenenfalls Dotierstoff(e)) können auf einen Träger aufgebracht werden. Geeignete Träger sind prinzipiell die im Folgenden genannten Adsorbentien und Katalysatorträger. Vorzugsweise ist das Trägermaterial ausgewählt unter Aktivkohle, Graphit, Ruß, Al₂O₃, SiO₂, TiO₂, ZrO₂, SiC, Silikaten, Zeolithen, Tonerden (z. B. Bentonite) und Kombinationen davon. Das Aufbringen wenigstens einer Metallkomponente und gegebenenfalls weiterer Komponenten auf ein Trägermaterial kann nach bekannten Verfahren erfolgen, z. B. durch (Co)-Präzipitation oder Imprägnieren. Die Entschwefelungsmittel können als Formkörper eingesetzt werden, z. B. in Form von gepressten Zylindern, Tabletten, Pastillen, Wagenrädern, Ringen, Sternen oder Strangpresslingen, wie Vollsträngen, polylobären Strängen, Hohlsträngen und Wabenkörpern oder anderen geometrischen Körpern. Ungeträgerte Entschwefelungsmittel können nach üblichen Verfahren geformt werden, z. B. durch Extrudieren, Tablettieren, etc. Die Form geträgerter Entschwefelungsmittel wird durch die Form des Trägers bestimmt.

Zur katalytischen Entschwefelung wird vorzugsweise ein Entschwefelungsmittel eingesetzt, das Kupfer und Zink in einem Atomverhältnis von 1 : 0,3 bis 1 : 10, vorzugsweise 1 : 0,5 bis 1 : 3, insbesondere 1 : 0,7 bis 1 : 1,5, enthält. Bevorzugt wird ein Entschwefelungsmittel eingesetzt, das 35 bis 45 Gew.-% Kupferoxid, 35 bis 45 Gew.-% Zinkoxid und 10 bis 30 Gew.-% Aluminiumoxid enthält. In einer speziellen Ausführungsform handelt es sich bei dem Entschwefelungsmittel um eine zum Einsatz als Hydrierungskatalysator in Schritt b) befähigte Komponente. Diesbezüglich wird auf die folgende Offenbarung zu Hydrierkatalysatoren der zuvor genannten Zusammensetzung und Verfahren zu ihrer Herstellung Bezug genommen.

In einer Ausgestaltung dieser Verfahrensvariante werden die glycerinhaltigen Ströme in wenigstens einer Entschwefelungszone mit dem Entschwefelungsmittel in Kontakt gebracht und anschließend in wenigstens einer Reaktionszone hydriert.

Es versteht sich für den Fachmann, dass die konkrete Ausgestaltung und Anordnung der Entschwefelungs- und Reaktionszone(n) in jeder bekannten Weise erfolgen kann. Es ist möglich, die Entschwefelungs- und Reaktionszone(n) räumlich voneinander getrennt anzuordnen, d. h. durch die apparative Ausgestaltung baulich voneinander zu separieren oder auch in einer oder mehreren gemeinsamen Entschwefelungs-/Hydrierzone(n) zu verwirklichen.

Das Kupfer-Zink-Entschwefelungsmittel kann z. B. durch ein übliches Fällungs- oder Copräzipitationsverfahren erhalten und in oxidierter als auch in reduzierter Form eingesetzt werden.

In einer besonderen Ausführungsform enthält das Kupfer-Zink-Entschwefelungsmittel mindestens Kupfer, Zink und Aluminium, wobei das Kupfer: Zink : Aluminium-Atomverhältnis im Bereich von 1 : 0,3 : 0,05 bis 1 : 10 : 2, vorzugsweise bei 1 : 0,6 : 0,3 bis 1 : 3 : 1 und insbesondere bei 1 : 0,7 : 0,5 bis 1 : 1,5 : 0,9 liegt.

Zur Überführung in die reduzierte Form ist es möglich, das Entschwefelungsmittel einer Wasserstoffreduktion zu unterwerfen. Diese wird bei etwa 150 bis 350 °C, vorzugsweise bei etwa 150 bis 250 °C, in Gegenwart von Wasserstoff durchgeführt, wobei der Wasserstoff durch ein Inertgas, wie z. B. Stickstoff, Argon, Methan, insbesondere Stickstoff, verdünnt wird, so dass der Wasserstoffgehalt 10 Vol.-% oder weniger, vorzugsweise 6 Vol.-% oder weniger, insbesondere 0,5 bis 4 Vol.-%, beträgt. Das so erhaltene Kupfer-Zink-Entschwefelungsmittel ("reduzierte Form") kann in dieser Form in die Entschwefelung eingesetzt werden.

In einer Ausführungsform wird die Entschwefelung des glycerinhaltigen Stroms an dem Kupfer-Zink-Entschwefelungsmittel in oxidierter Form ohne Zusatz von Wasserstoff durchgeführt.

In einer weiteren Ausführungsform wird die Entschwefelung des glycerinhaltigen Stroms an dem Kupfer-Zink-Entschwefelungsmittel in oxidierter Form in Gegenwart von Wasserstoff durchgeführt.

In einer weiteren Ausführungsform wird die Entschwefelung des glycerinhaltigen Stroms an dem Kupfer-Zink-Entschwefelungsmittel in reduzierter Form ohne Zusatz von Wasserstoff durchgeführt.

In einer weiteren Ausführungsform wird die Entschwefelung des glycerinhaltigen Stroms an dem Kupfer-Zink-Entschwefelungsmittel in reduzierter Form in Gegenwart von Wasserstoff durchgeführt.

Üblicherweise wird die Entschwefelung in einem Temperaturbereich von 40 bis 200 °C, besonders bei 50 bis 180 °C, insbesondere bei 60 bis 160 °C, vorzugsweise bei 70 bis 120 °C, bei einem Druck von 1 bis 40 bar, besonders bei 1 bis 32 bar, vorzugsweise bei 1,5 bis 5 bar, insbesondere bei 2,0 bis 4,5 bar, durchgeführt. Die Entschwefelung kann in Gegenwart von Inertgasen, wie z. B. Stickstoff, Argon oder Methan, durchgeführt werden. In der Regel wird jedoch die Entschwefelung ohne Zugabe von Inertgasen durchgeführt.

Üblicherweise setzt man - soweit gewünscht - hierbei Wasserstoff mit einer Reinheit von ≥ 99,8 Vol.-%, insbesondere von ≥ 99,9 Vol.-%, vorzugsweise von ≥ 99,95 Vol.-%, ein. Diese Reinheitsgrade gelten analog für den Wasserstoff, der bei den gegebenenfalls durchgeführten Aktivierungen der Katalysatoren verwendet wird.

Üblicherweise liegt das Gewichtsverhältnis von glycerinhaltigem Strom zu Wasserstoff im Bereich von 40000 : 1 bis 1000 : 1, besonders im Bereich von 38000 : 1 bis 5000 : 1, insbesondere im Bereich von 37000 : 1 bis 15000 : 1, vorzugsweise im Bereich von 36000 : 1 bis 25000 : 1, im Speziellen im Bereich von 35000 : 1 bis 30000 : 1.

Der so entschwefelte glycerinhaltige Strom hat im Allgemeinen einen Gehalt von schwefelhaltigen Verunreinigungen, speziell von aromatischen Schwefelverbindungen, von höchstens 70 ppb, vorzugsweise von höchstens 50 ppb, und der Gesamtschwefelgehalt liegt bei insgesamt ≤ 200 ppb, vorzugsweise ≤ 150 ppb, insbesondere ≤ 100 ppb.

Die voranstehend beschriebenen Entschwefelungsmittel ermöglichen es auch, Chlor, Arsen und/oder Phosphor bzw. entsprechende chlor-, arsen- und/oder phosphorhaltige Verbindungen aus dem aromatischen Kohlenwasserstoff, oder dem Gemisch von aromatischen Kohlenwasserstoffen, zu reduzieren oder zu entfernen.

In einer weiteren Ausführung wird der glycerinhaltige Strom in Schritt a) zur Entfernung von Komponenten, welche die katalytische Hydrierung beeinträchtigen, mit wenigstens einem Adsorptionsmittel in Kontakt gebracht.

Die Adsorptionsmittel haben vorzugsweise eine spezifische Oberfläche, bestimmt nach BET, im Bereich von 10 bis 2000 m²/g, besonders bevorzugt im Bereich von 10 bis 1500 m²/g, insbesondere im Bereich von 10 bis 400 m²/g, speziell im Bereich von 60 bis 250 m²/g.

Geeignete Adsorptionsmittel sind z. B. aktive Aluminiumoxide. Ihre Herstellung erfolgt z. B. ausgehend von Aluminiumhydroxid, welches durch übliche Fällungsverfahren aus Aluminiumsalzlösungen erhältlich ist. Für das erfindungsgemäße Verfahren geeignete aktive Aluminiumoxide sind auch ausgehend von Aluminiumhydroxid-Gelen erhältlich. Zur Herstellung solcher Gele kann z. B. gefälltes Aluminiumhydroxid nach üblichen Aufarbeitungsschritten, wie Filtern, Waschen und Trocknen, aktiviert und anschließend gegebenenfalls vermahlen oder agglomeriert werden. Gewünschtenfalls kann man das resultierende Aluminiumoxid anschließend noch einem Formgebungsverfahren, wie Extrusion, Granulation, Tablettierung etc. unterwerfen. Als Adsorptionsmittel eignen sich vorzugsweise die Selexsorb TM-Typen der Firma Alcoa.

Geeignete Adsorptionsmittel sind weiterhin aluminiumoxidhaltige Feststoffe. Hierzu zählen z. B. die so genannten Tonerden, die als Hauptbestandteil ebenfalls Aluminiumoxide aufweisen.

Des Weiteren geeignete Adsorptionsmittel sind Aluminiumphosphate.

Weiterhin geeignete Adsorptionsmittel sind Siliciumdioxide, die z. B. durch Entwässerung und Aktivierung von Kieselgelen erhältlich sind. Ein weiteres Verfahren zur Herstellung von Siliciumdioxid ist die Flammhydrolyse von Siliciumtetrachlorid, wobei durch geeignete Variationen der Reaktionsparameter, wie z. B. der stöchiometrischen Zusammensetzung des Eduktgemisches und der Temperatur, die gewünschten Oberflächeneigenschaften des resultierenden Siliciumdioxids in weiten Bereichen variiert werden können.

Weiterhin geeignete Adsorptionsmittel sind Kieselgure, die ebenfalls als Hauptbestandteil Siliciumdioxide aufweisen. Dazu zählt z. B. die aus Kieselsedimenten gewonnene Diatomeenerde.

Weiterhin geeignete Adsorptionsmittel sind Titandioxide und Zirkoniumdioxide, wie sie z. B. in Römpp, Chemie-Lexikon, 9. Aufl. (Paperback), Bd. 6, S. 4629f. und S. 5156f. und der dort zitierten Literatur beschrieben sind. Hierauf wird hier in vollem Umfang Bezug genommen.

Weiterhin geeignete Adsorptionsmittel sind Phosphate, insbesondere kondensierte Phosphate, wie z. B. Schmelz- oder Glühphosphate, die eine große aktive Oberfläche aufweisen. Geeignete Phosphate sind z. B. in Römpp, Chemie-Lexikon, 9. Aufl. (Paperback), Bd. 4, S. 3376f. und der dort zitierten Literatur beschrieben. Hierauf wird hier in vollem Umfang Bezug genommen.

Weiterhin geeignete Adsorptionsmittel sind kohlenstoffhaltige Adsorbentien, bevorzugt Aktivkohle. Unter Aktivkohle versteht man hierbei im Allgemeinen Kohlenstoff mit poröser Struktur und hoher innerer Oberfläche. Zur Herstellung von Aktivkohle werden pflanzliche, tierische und/oder mineralische kohlenstoffhaltige Rohstoffe, z. B. mit Dehydratisierungsmitteln, wie Zinkchlorid oder Phosphorsäure, erhitzt oder durch trockene Destillation verkohlt und anschließend oxidativ aktiviert. Dazu kann man z. B. das verkohlte Material bei erhöhten Temperaturen von etwa 700 bis 1000 °C mit Wasserdampf, Kohlendioxid und/oder Gemischen davon behandeln.

Möglich ist auch ein Einsatz von Ionenaustauschern und/oder Adsorberharzen.

Die Adsorptionsmittel sind vorzugsweise unter Titandioxiden, Zirkoniumdioxiden, Siliciumdioxiden, Kieselgur, Aluminiumoxiden, aluminiumoxidhaltigen Feststoffen, Aluminiumphosphaten, natürlichen und synthetischen Aluminiumsilicaten, Phosphaten, kohlenstoffhaltigen Adsorbentien und Mischungen davon ausgewählt.

Die Adsorptionsmittel weisen im Allgemeinen eine spezifische Oberfläche, bestimmt nach BET, im Bereich von etwa 10 bis 2000 m²/g, insbesondere im Bereich von 10 bis 1500 m²/g und speziell im Bereich von 20 bis 600 m²/g, auf.

Zur adsorptiven Entfernung von unerwünschten Komponenten, insbesondere von Komponenten, welche die katalytische Hydrierung beeinträchtigen, wird der glycerinhaltige Strom in Schritt a) in einer Adsorptionszone mit wenigstens einem Adsorptionsmittel in Kontakt gebracht.

In einer speziellen Ausführungsform wird ein Adsorptionsmittel eingesetzt, welches wenigstens eine auch zum Einsatz als Hydrierungskatalysator in Schritt b) befähigte Komponente enthält. Auf die im Folgenden genauer beschriebenen Hydrierungskatalysatoren wird hier im vollen Umfang Bezug genommen. Zum Einsatz als Adsorptionsmittel eignen sich auch Kombinationen aus zwei oder mehr als zwei Adsorptionsmitteln. Dabei können sowohl ausschließlich auch als Hydrierungskatalysatoren befähigte Komponenten, ausschließlich nicht als Hydrierungskatalysatoren geeignete Adsorptionsmittel sowie Kombinationen davon eingesetzt werden.

In einer bevorzugten Ausführungsform setzt man als Adsorptionsmittel und als Hydrierungskatalysator die gleiche Komponente ein. Gegebenenfalls setzt man hierbei zusätzlich ein oder mehrere weitere, von dem Hydrierungskatalysator verschiedene herkömmliche Adsorptionsmittel, wie zuvor beschrieben, ein.

In einer Ausgestaltung des Verfahrens werden die glycerinhaltigen Ströme in wenigstens einer Adsorptionszone mit dem Adsorptionsmittel in Kontakt gebracht und anschließend in wenigstens einer Reaktionszone hydriert.

Es versteht sich für den Fachmann, dass die konkrete Ausgestaltung und Anordnung der Adsorptions- und Reaktionszone(n) in jeder bekannten Weise erfolgen kann. Es ist bevorzugt, die Adsorptions- und Reaktionszone(n) räumlich voneinander getrennt anzuordnen, d. h. durch die apparative Ausgestaltung baulich voneinander zu separieren.

Sofern man unterschiedliche Adsorptionsmittel verwendet, kann man z. B. eine erste Adsorptionszone in einem ersten Reaktor vorsehen, die ein erstes Adsorptionsmittel enthält, und separat, also apparativ getrennt davon, z. B. in einem zweiten Reaktor, eine zweite Adsorptionszone, die ein zweites Adsorptionsmittel enthält. Hierbei kann das erste und/oder das zweite Adsorptionsmittel wenigstens eine zum Einsatz als Hydrierungskatalysators befähigte Komponente enthalten.

In einer weiteren Ausführungsform setzt man ein herkömmliches Adsorptionsmittel zusammen mit einem zur Hydrierung befähigten Adsorptionsmittel in einer einzigen Adsorptionszone ein, z. B. in übereinander geschichteter Form, gemischt in Form einer statistischen Verteilung oder in Form einer Gradientenschüttung. Die Verwendung in gemischter Form erlaubt gegebenenfalls eine bessere Kontrolle der Temperatur. Im Fall einer Gradientenschüttung können lineare und nicht-lineare Gradienten verwendet werden. Es kann hierbei vorteilhaft sein, die Verteilung innerhalb der Schüttung derart vorzunehmen, dass der zu hydrierende glycerinhaltige Strom zunächst mit dem herkömmlichen Adsorptionsmittel in Kontakt gebracht wird, bevor er mit dem zur Hydrierung befähigten Adsorptionsmittel in Kontakt gebracht wird.

Vorteilhafterweise wird man wenigstens zwei Adsorptionszonen so anordnen, dass der zu hydrierende glycerinhaltige Strom in der ersten Adsorptionszone mit einem herkömmlichen Adsorptionsmittel in Kontakt gebracht wird und in der zweiten Adsorptionszone mit einem Adsorptionsmittel, das wenigstens eine zum Einsatz als Hydrierungskatalysator befähigte Komponente enthält, in Kontakt gebracht wird.

Die in Schritt a) des erfindungsgemäßen Verfahrens bereitgestellten glycerinhaltigen Ströme stammen bevorzugt aus der Herstellung von Biodiesel. Im Rahmen der vorliegenden Erfindung wird unter "Biodiesel" ein Gemisch aus Fettsäuremonoalkylestern verstanden, das aus biogenen öl- und/oder fetthaltigen Ausgangsgemischen gewonnen und in Dieselmotoren als Kraftstoff eingesetzt werden kann.

Zur Bereitstellung des glycerinhaltigen Stroms eigenen sich prinzipiell alle verfügbaren biogenen öl- und/oder fetthaltigen Ausgangsgemische. Öle und Fette sind allgemein feste, halbfeste oder flüssige Fettsäuretriglyceride, insbesondere aus pflanzlichen und tierischen Quellen, die chemisch im Wesentlichen aus Glycerinestern höherer Fettsäuren bestehen. Geeignete höhere Fettsäuren sind gesättigte oder ein- oder mehrfach ungesättigte Fettsäuren mit vorzugsweise 8 bis 40, besonders bevorzugt 12 bis 30 Kohlenstoffatomen. Dazu zählen z. B. n-Nonansäure, n-Decansäure, n-Undecansäure, n-Tridecansäure, Myristinsäure, Pentadecansäure, Palmitinsäure, Margarinsäure, Nonadecansäure, Arachinsäure, Behensäure, Lignocerinsäure, Cerotinsäure, Melissinsäure, Palmitoleinsäure, Oleinsäure, Linolsäure, Linolensäure, Stearinsäure, Elaostearinsäure, etc.

Pflanzliche Fette und Öle basieren im Wesentlichen auf Fettsäuren mit gerader Kohlenstoffatomanzahl, wohingegen tierische Fette und Öle auch Fettsäuren mit ungerader Kohlenstoffatomanzahl in freier oder als Triglyceridester gebundener Form enthalten können. Die in pflanzlichen Fetten und Ölen vorkommenden ungesättigten Fettsäuren liegen in der cis-Form vor, während tierische Fettsäuren häufig transkonfiguriert sind.

Zur Bereitstellung des glycerinhaltigen Stroms in Schritt a) können prinzipiell gebrauchte oder ungebrauchte, ungereinigte oder gereinigte pflanzliche, tierische oder technische Öle oder Fette oder Gemische davon eingesetzt werden. Diese können Anteile weiterer Inhaltsstoffe, z. B. freie Fettsäuren, enthalten. Der Anteil freier Fettsäuren beträgt im Allgemeinen 0 % bis 50 %, z. B. 0,1 bis 20 %, des zur Umesterung der Fettsäuretriglyceride eingesetzten Ausgangsgemischs. Freie Fettsäuren können gewünschtenfalls vor oder nach der Umesterung der Fettsäuretriglyceride entfernt werden. Salze dieser Fettsäuren (z. B. die Alkalisalze) können zuvor durch Ansäuern mit einer starken Säure, z. B. HCl, in die freie Säure überführt werden. Die Abtrennung der freien Fettsäuren gelingt z. B. durch Zentrifugieren. Vorzugsweise werden die in dem Ausgangsgemisch enthaltenen freien Fettsäuren ebenfalls in die Alkylester überführt. Dies kann vor, während oder nach der Umesterung der Fettsäuretriglyceride erfolgen.

Zur Bereitstellung des glycerinhaltigen Stroms in Schritt a) geeignete gebrauchte Fette und Öle sind fett- und/oder ölhaltige Komponenten, die nach ihrer Gewinnung aus entsprechenden biogenen Ausgangsmaterialien zunächst zu anderen Zwecken, z. B. zu technischen Zwecken oder Zwecken der Nahrungsmittelherstellung, verwendet wurden und die infolge dieser Verwendung chemisch modifiziert oder unmodifiziert sind oder zusätzliche Inhaltsstoffe, die insbesondere im Zusammenhang mit dieser Verwendung stehen, aufweisen können. Diese können gewünschtenfalls vor dem Einsatz zur Bereitstellung des glycerinhaltigen Stroms durch Umesterung zumindest teilweise entfernt werden. Zur Bereitstellung des glycerinhaltigen Stroms in Schritt a) geeignete ungebrauchte Fette und Öle sind fett- oder ölhaltige Komponenten, die nach ihrer Gewinnung aus den entsprechenden pflanzlichen oder tierischen Ausgangsmaterialien noch keinem anderen Zweck zugeführt wurden und die daher nur Inhaltsstoffe aufweisen, die aus den Ausgangsmaterialien stammen bzw. die mit der Gewinnung aus den Ausgangsmaterialien im Zusammenhang stehen. Auch aus diesen Ausgangsmaterialien können andere Inhaltsstoffe als Fettsäuretriglyceride (und gegebenenfalls freie Fettsäuren) gewünschtenfalls vor dem Einsatz zur Bereitstellung des glycerinhaltigen Stroms durch Umesterung zumindest teilweise entfernt werden.

Zur Aufreinigung und/oder Anreicherung können die ungebrauchten oder gebrauchten Fette oder Öle einer Entfernung von ungewünschten Inhaltsstoffen, wie Lecithinen, Kohlenhydraten, Proteinen, Ölschlamm, Wasser, etc. unterzogen werden.

Pflanzliche Öle und Fette sind solche, die zum überwiegenden Teil aus pflanzlichen Ausgangsmaterialien wie Samen, Wurzeln, Blättern oder anderen geeigneten Pflanzenteilen stammen. Tierische Fette oder Öle stammen zum überwiegenden Teil aus tierischen Ausgangsmaterialien, wie tierischen Organen, Geweben oder anderen Körperteilen oder Körperflüssigkeiten wie Milch. Technische Öle und Fette sind solche, die insbesondere aus tierischen oder pflanzlichen Ausgangsmaterialien gewonnen und für technische Zwecke aufbereitet wurden. Die erfindungsgemäß eingesetzten gebrauchten oder ungebrauchten, ungereinigten oder gereinigten Öle und/oder Fette sind insbesondere ausgewählt aus der Gruppe, bestehend aus Soapstock, Brown Grease, Yellow Grease, technischem Talg, technischem Schmalz, Frittierölen, Tierfett, Speisetalg, pflanzlichen Rohölen, tierischen Rohölen oder -fetten oder Gemischen davon.

Unter "Soapstock" wird ein bei der Verarbeitung von pflanzlichen Ölen anfallendes Nebenprodukt verstanden, insbesondere ein Nebenprodukt von Speiseöl-Raffinerien auf der Basis von Soja-, Rüb- oder Sonnenblumenöl. Soapstock weist einen Anteil von freien Fettsäuren von etwa 50 % bis 80 % auf.

Unter "Brown Grease" wird ein tierfetthaltiges Abfallprodukt verstanden, das einen Anteil von freien Fettsäuren von über 15 % bis 40 % aufweist. "Yellow Grease" enthält etwa 5 % bis 15 % freie Fettsäuren.

Unter "technischem Talg" und "technischem Schmalz" werden tierische Fette verstanden, die für technische Zwecke hergestellt und nach dem Trocken- oder Nassschmelzverfahren beispielsweise aus Schlachtabfällen gewonnen werden. Technische Talge werden nach ihrer Säurezahl bewertet und gehandelt, wobei der Gehalt an freien Fettsäuren je nach Herkunft z. B. zwischen 1 und 20 Gew.-%, wie beispielsweise im Bereich von 1 bis 15 Gew.-% liegt. Der Gehalt an freien Fettsäuren kann jedoch auch mehr als 20 Gew.-% betragen.

Zu den "Tierfetten" gehören insbesondere bei der Verwertung von Geflügel-, Rinder-, Schweine-, Fisch- und Meeressäuger-Körpern abfallende fetthaltige Produkte, beispielsweise Solarstearin, ein fester Rückstand, der nach dem Auspressen von Schmalzöl aus Schweineschmalz verbleibt.

Die Bereitstellung des glycerinhaltigen Stroms in Schritt a) erfolgt vorzugsweise aus pflanzlichen Rohölen als Ausgangsmaterial. Dabei kann man von ungereinigten pflanzlichen Rohölen ausgehen, d. h. von flüssigen oder festen Zusammensetzungen, die aus pflanzlichen Ausgangsmaterialien z. B. durch Pressen gewonnen werden, wobei sie keine andere Behandlung erfahren haben als Absetzen in allgemein üblichen Zeiträumen und Abschleudern oder Filtrieren, bei dem zur Trennung des Öls von festen Bestandteilen nur mechanische Kräfte wie Schwerkraft, Fliehkraft oder Druck eingesetzt werden. Solche ungereinigten pflanzlichen Rohöle können auch durch Extraktion gewonnene pflanzliche Öle sein, wenn sich deren Eigenschaften von den entsprechenden, mittels Pressen gewonnenen pflanzlichen Ölen nicht oder nur unwesentlich unterscheiden. Der Anteil freier Fettsäuren in ungereinigten pflanzlichen Fetten und Ölen ist unterschiedlich und liegt z. B. bei etwa 0 bis 20 %, wie z. B. 0,1 bis 15 %.

Selbstverständlich können die pflanzlichen Öle vor ihrem Einsatz zur Umesterung einem oder mehreren Aufarbeitungsschritten unterzogen werden, wie sie im Folgenden noch genauer beschrieben sind. So können auch gereinigte pflanzliche Öle, beispielsweise Raffinate oder Halbraffinate, der vorstehend genannten pflanzlichen Öle als Ausgangsmaterialien eingesetzt werden.

Vorzugsweise wird zur Bereitstellung des glycerinhaltigen Stroms in Schritt a) ein pflanzliches Öl bzw. Fett eingesetzt, das vorzugsweise ausgewählt ist unter Rapsöl, Palmöl, Rüböl, Sojaöl, Sonnenblumenöl, Maiskeimöl, Baumwollsaatöl, Palmkern- und Kokosfett und Mischungen davon. Besonders bevorzugt wird Rapsöl oder ein rapsölhaltiges Gemisch eingesetzt.

Geeignet zur Bereitstellung des glycerinhaltigen Stroms in Schritt a) ist auch tierisches Öl bzw. Fett, das vorzugsweise ausgewählt ist unter Milchfett, Wollfett, Rindertalg, Schweineschmalz, Fischölen, Fischtran, etc. und Mischungen davon. Auch diese tierischen Fette oder Öle können vor ihrem Einsatz zur Umesterung einem oder mehreren Aufarbeitungsschritten unterzogen werden, wie sie im Folgenden noch genauer beschrieben sind.

Vorzugsweise umfasst die Bereitstellung des glycerinhaltigen Stroms in Schritt a) die folgenden Schritte:
a1) Bereitstellen eines biogenen fett- und/oder ölhaltigen Ausgangsgemisches,
a2) Umestern der in dem Ausgangsgemisch enthaltenen Fettsäuretriglyceride mit wenigstens einem C₁-C₉-Monoalkohol und gegebenenfalls Verestern der in dem Ausgangsgemisch enthaltenen freien Fettsäuren unter Bildung eines Veresterungsgemisches,
a3) Auftrennen des Veresterungsgemisches unter Erhalt wenigstens einer an Biodiesel angereicherten Fraktion und wenigstens einer an bei der Veresterung freigesetztem Glycerin angereicherten Fraktion,
a4) gegebenenfalls Aufreinigen der Glycerin angereicherten Fraktion.

### Schritt a1)

Die Bereitstellung des biogenen fett- und/oder ölhaltigen Ausgangsgemisches in Schritt a1) umfasst in einer bevorzugten Ausführungsform wenigstens einen Reinigungsschritt. Zur Reinigung kann das fett- und/oder ölhaltige Ausgangsgemisch wenigstens einem üblicherweise verwendeten Reinigungsverfahren für Fette und Öle, wie Klären, Filtration, Behandlung mit Bleicherden oder Behandlung mit Säuren oder Alkali zur Abtrennung störender Verunreinigungen wie Proteine, Phosphatide und Schleimstoffe sowie einer Kombination von wenigstens zwei dieser Reinigungsschritte unterzogen werden.

### Schritt a2)

Zur Umesterung der Fettsäuretriglyceride wird vorzugsweise wenigstens ein C₁-C₉-Monoalkohol, insbesondere wenigstens ein C₁-C₄-Monoalkohol eingesetzt. Bevorzugt ist der Einsatz von Methanol oder Ethanol.

Die Umesterung der Fettsäuretriglyceride kann sauer oder vorzugsweise basisch katalysiert erfolgen. Geeignete Säuren sind beispielsweise Mineralsäuren wie HCl, H₂SO₄ oder H₃PO₄.

Bevorzugt wird als Katalysator wenigstens eine Base eingesetzt. Diese ist vorzugsweise ausgewählt unter Alkalihydroxiden, wie NaOH und KOH, Erdalkalihydroxiden, wie Ca(OH)₂, Alkali- und Erdalkali-C₁-C₆-alkanolaten, wie NaOCH₃, KOCH₃, Na(OCH₂CH₂) und Ca(OCH₂CH₂)₂ und Mischungen davon. Besonders bevorzugt verwendet man NaOH, KOH oder NaOCH₃, ganz besonders bevorzugt NaOCH₃.

Die Menge der eingesetzten Base liegt üblicherweise im Bereich von 0,1 bis 10 Gew.-%, insbesondere von 0,2 bis 5 Gew.-%, bezogen auf die eingesetzte Menge an Fettsäuretriglyceriden.

Die Base wird bevorzugt in Form einer wässrigen oder alkoholischen, besonders bevorzugt alkoholischen, Lösung eingesetzt. Vorteilhafterweise verwendet man als Lösungsmittel für die Base das bereits für die Alkoholyse der Triglyceride eingesetzte Lösungsmittel. Bevorzugt wird zur Umesterung eine NaOCH₃-Lösung in Methanol eingesetzt.

Die Umesterung erfolgt vorzugsweise bei einer Temperatur von etwa 20 bis 150 °C, insbesondere 30 bis 95 °C.

Die Umesterung erfolgt in dafür üblichen, dem Fachmann bekannten Vorrichtungen. Nach einer geeigneten Ausführung erfolgt die Umesterung kontinuierlich. Vorzugsweise erfolgt die Umesterung in wenigstens einer Kolonne, wobei das erhaltene Umesterungsgemisch gleichzeitig einer Auftrennung unterzogen wird. Dabei wird im Allgemeinen eine höhersiedende Phase erhalten, die an dem basischen Katalysator, an nicht umgesetztem Monoalkohol und dem bei der Umesterung entstandenen Glycerin angereichert ist und eine niedriger siedende Phase erhalten, die an dem Umesterungsprodukt angereichert ist. Sofern das Umesterungsprodukt noch nicht umgeesterte Triglyceride enthält, können diese ebenfalls abgetrennt und einer erneuten Umesterung in der ersten oder einer weiteren Umesterungsstufe unterzogen werden.

Anschließend wird das letzte Umesterungsgemisch in eine Trocknungsanlage überführt, wobei nochmals Restmengen an Wasser entfernt werden. Nach der Trocknung in der Trocknungsvorrichtung liegt das gewünschte Endprodukt Biodiesel in gereinigter Form vor und kann direkt als Kraftstoff eingesetzt werden.

Sofern das zur Bereitstellung des glycerinhaltigen Stroms in Schritt a) eingesetzte fett- und/oder ölhaltige Ausgangsgemisch freie Fettsäuren enthält, können diese vorzugsweise einer Veresterung zur Umwandlung in Biodiesel-taugliche Ester unterzogen werden.

Die freien Fettsäuren werden vorzugsweise mit dem gleichen C₁-C₉-Monoalkohol umgeestert, der zur Umesterung der Fettsäuretriglyceride eingesetzt wurde. Die Veresterung freier Fettsäuren kann vor, während oder nach der Umesterung der Fettsäuretriglyceride erfolgen. In einer bevorzugten Ausführung erfolgt die Veresterung freier Fettsäuren vor der Umesterung der Fettsäuretriglyceride.

Die Veresterung der freien Fettsäuren kann basisch oder vorzugsweise sauer katalysiert erfolgen. Geeignete Säuren sind die zuvor genannten Mineralsäuren, wie HCl, H₂SO₄ oder H₃PO₄, p-Toluolsulfonsäure, etc. Die Veresterung erfolgt vorzugsweise bei einer Temperatur von etwa 20 bis 95 °C, insbesondere 40 bis 80 °C.

Die Veresterung erfolgt in dafür üblichen, dem Fachmann bekannten Vorrichtungen. Dazu zählen Rührkessel und/oder Kolonnen, die gewünschtenfalls zu Kaskaden geschaltet sind. Bevorzugt erfolgt die Veresterung der freien Fettsäuren in wenigstens einer als Kolonne ausgeführten Veresterungseinrichtung, wobei das erhaltene Veresterungsgemisch gleichzeitig einer Auftrennung unterzogen wird. In einer geeigneten Ausführungsform erfolgt die Veresterung in Gegenwart eines Schleppmittels zur Erleichterung der Trennung.

### Schritt a3)

Während oder im Anschluss an die Umesterung und/oder Veresterung wird das Veresterungsgemisch einer Auftrennung unter Erhalt wenigstens einer an C₁-C₉-Monoalkylestern angereicherten Fraktion und wenigstens einer an bei der Umesterung freigesetztem Glycerin angereicherten Fraktion unterzogen. Die Auftrennung erfolgt vorzugsweise destillativ nach üblichen, dem Fachmann bekannten Verfahren. Geeignete Destillationsvorrichtungen sind die zuvor genannten.

### Schritt a4)

Die nach Auftrennen des Veresterungsgemisches in Schritt a3) erhaltene an Glycerin angereicherte Fraktion kann gegebenenfalls wenigstens einem Aufarbeitungsschritt unterzogen werden. Dazu zählt beispielsweise die Entfernung von unerwünschten Komponenten, wie Salzen, sowie von Komponenten, welche die katalytische Hydrierung beeinträchtigen oder die Abtrennung von Wasser und, falls vorhanden, organischem Lösungsmittel. Auf die vorherigen Ausführungen zu diesen Aufarbeitungsschritten wird in vollem Umfang Bezug genommen.

Bei den in dem erfindungsgemäßen Verfahren eingesetzten heterogenen Hydrierungskatalysatoren kann es sich um Vollkatalysatoren oder geträgerte Katalysatoren handeln. Sie können in Form von einheitlich zusammengesetzten Katalysatoren, imprägnierten Katalysatoren, beschichteten Katalysatoren und Fällungskatalysatoren eingesetzt werden.

Prinzipiell eignen sich eine Vielzahl von kupferhaltigen Katalysatoren, die zusätzlich wenigstens ein weiteres Element der I., II.,III., IV oder V. Hauptgruppe, der I., II., IV., V., VI., VII. oder VIII. Nebengruppe sowie der Lanthanide enthalten können (IUPAC: Gruppen 1 bis 15 und die Lanthanide), insbesondere Ca, Mg, Al, La, Ti, Zr, Cr, Mo, W, Mn, Ni, Co, Zn und Kombinationen davon.

Eine spezielle Ausführungsform von Katalysatoren, die sich besonders vorteilhaft für einen Einsatz in dem erfindungsgemäßen Verfahren eignen, sind Skelett- oder Metallschwamm-Katalysatoren, die als "Raney-Katalysatoren" bezeichnet werden. Dazu zählen speziell Raney-Kupfer sowie kupferhaltige Metalllegierungen in Form eines Raney-Katalysators. Bevorzugt sind Raney-Katalysatoren, deren Metallkomponente zu wenigstens 95 %, insbesondere zu wenigstens 99 %, aus Kupfer besteht. Verfahren zur Herstellung von Raney-Katalysatoren sind dem Fachmann bekannt und z. B. in der DE-A-43 35 360, DE-A-43 45 265, DE-A-44 46 907 und EP-A-842 699 beschrieben. Raney-Kupfer kann in an sich bekannter Weise durch Behandeln von Kupfer-Aluminium-Legierungen mit Alkalihydroxiden hergestellt werden. Ein für den Einsatz in dem erfindungsgemäßen Verfahren geeigneter Raney-Katalysator ist z. B. erhältlich durch Herstellen einer Mischung aus wenigstens einer kupferhaltigen Katalysatorlegierung und wenigstens einem Bindemittel, wobei die Katalysatorlegierung Kupfer und gegebenenfalls wenigstens ein weiteres katalytisch aktives Katalysatormetall und eine auslaugbare Legierungskomponente enthält, gegebenenfalls unter Zugabe von Befeuchtungsmitteln und/oder Zusatzstoffen wie Verformungshilfsmitteln, Gleitmitteln, Plastifizierungsmitteln und/oder Porenbildnern, Homogenisieren dieser Mischung und Verformen zu dem gewünschten Formkörper, Kalzinieren des Formkörpers und Aktivieren der so erhaltenen Katalysatorvorstufe durch teilweises oder vollständiges Auslaugen der auslaugbaren Legierungskomponente sowie gegebenenfalls abschließendes Waschen des fertigen Katalysators.

Eine weitere spezielle Ausführungsform von Katalysatoren, die sich besonders vorteilhaft für einen Einsatz in dem erfindungsgemäßen Verfahren eignen, sind Katalysatoren, die Kupfer in oxidischer Form sowie gegebenenfalls zusätzlich in elementarer Form enthalten. Der in Schritt b) eingesetzte Hydrierkatalysator enthält dann vorzugsweise wenigstens 23 Gew.-%, besonders bevorzugt wenigstens 35 Gew.-%, Kupfer in oxidischer und/oder elementarer Form, bezogen auf das Gesamtgewicht des Katalysators.

Ein häufig angewandtes Verfahren zur Herstellung solcher Katalysatoren besteht in der Tränkung von Trägermaterialien mit Lösungen der Katalysatorkomponenten, die anschließend durch thermische Behandlung, Zersetzung oder Reduktion in den katalytisch aktiven Zustand überführt werden.

Ein weiteres geeignetes Verfahren zur Herstellung von Katalysatoren umfasst die Fällung (Präzipitation) einer Katalysator-Komponente oder die Co-Fällung von zwei oder mehr als zwei Katalysator-Komponenten. So kann zur Herstellung eines Katalysatorformkörpers eine Kupferverbindung, gegebenenfalls wenigstens eine weitere Metallverbindung und/oder ein Additiv gefällt und anschließend einer Trocknung, Kalzination und Formung unterzogen werden. Die Fällung kann in Gegenwart eines Trägermaterials durchgeführt werden. Geeignete Ausgangsmaterialien für die Fällung sind Metallsalze und Metallkomplexe. Als Kupferverbindungen für die Fällung können prinzipiell alle bekannten Cu(I) und/oder Cu(II)-Salze eingesetzt werden, die in den zur Auftragung auf den Träger eingesetzten Lösungsmitteln löslich sind. Dazu zählen z. B. Nitrate, Carbonate, Acetate, Oxalate oder Ammoniumkomplexe. In einer bevorzugten Ausführung wird Kupfernitrat eingesetzt. Die katalytische aktive Komponente des Katalysators kann, abgesehen von einer Kupferverbindung, weitere Elemente als Additive aufweisen, z. B. Metalle, Nichtmetalle und deren Verbindungen. Dabei handelt es sich vorzugsweise um Metalle der Gruppen 4 bis 15 und die Lanthanide. Besonders bevorzugte Metalle sind La, Ti, Zr, Cu, Mo, W, Mn, Re, Co, Ni, Cu, Ag, Au, Zn, Sn, Pb, As, Sb und Bi. Vorzugsweise wird zur Fällung ein wässriges Medium eingesetzt.

Geeignete wässrige Medien sind Substanzen oder Mischungen, die unter den Verfahrensbedingungen flüssig sind und die wenigstens 10 Gew.-%, besonders bevorzugt wenigstens 30 Gew.-%, insbesondere wenigstens 50 Gew.-%, Wasser enthalten. Der von Wasser verschiedene Anteil ist vorzugsweise ausgewählt unter anorganischen oder organischen Verbindungen, die wenigstens teilweise in Wasser löslich oder wenigstens teilweise mit Wasser mischbar sind. Beispielsweise sind die von Wasser verschiedenen Verbindungen ausgewählt unter organischen Lösungsmitteln, wie C₁-C₂₀-Alkanolen, insbesondere Methanol, Ethanol, n-Propanol, Isopropanol, n-Butanol, sec.-Butanol, tert.-Butanol, Penthanolen und Hexanolen, C₄-C₈-Cycloalkylethern, wie Tetrahydrofuranen, Pyranen, Dioxanen und Trioxanen, C₁-C₁₂-Dialkylethern, wie Dimethylether, Dibutylether und Methylbutylether. Das wässrige Medium enthält vorzugsweise weniger als 40 Gew.-%, besonders bevorzugt weniger als 30 Gew.-% und insbesondere weniger als 20 Gew.-% organische Lösungsmittel. In einer bevorzugten Ausführung des erfindungsgemäßen Verfahrens ist das wässrige Medium im Wesentlichen frei von organischen Lösungsmitteln.

Die Fällung kann durch bekannte Verfahren, z. B. Kühlen einer gesättigten Lösung, Zugabe eines Fällungsmittels, etc. induziert werden. Geeignete Fällungsmittel sind z. B. Säuren, Basen, Reduktionsmittel, etc.

Die Fällung kann durch Zugabe einer Säure oder einer Base zu dem wässrigen Medium induziert werden, das die Kupferverbindung und gegebenenfalls weitere Verbindungen enthält. Geeignete Säuren sind Mineralsäuren, wie HCl, H₂SO₄ und H₃PO₄. Die Base ist vorzugsweise ausgewählt unter Metalloxiden, Metallhydroxiden, insbesondere Alkalimetallhydroxiden, wie Natriumhydroxid und Kaliumhydroxid, Metallcarbonaten, insbesondere Alkalimetall- und Erdalkalimetallcarbonaten, wie Lithiumcarbonat, Natriumcarbonat, Kaliumcarbonat, Magnesiumcarbonat und Calciumcarbonat, Stickstoffbasen, insbesondere Ammoniak sowie primären, sekundären und tertiären Aminen.

Beispiele geeigneter Reduktionsmittel sind Carbonsäuren, wie Ameisensäure, Citronensäure, Milchsäure, Weinsäure und insbesondere Salze von Carbonsäuren, vorzugsweise die Alkalimetall-, Erdalkalimetall-, Ammonium- und C₁-C₁₀-Alkylammoniumsalze, phosphorige oder hypophosphorige Säure, die Salze von phosphoriger oder hypophosphoriger Säure, insbesondere die Alkalimetall- oder Erdalkalimetallsalze, C₁-C₁₀-Alkanole, wie Methanol, Ethanol und Isopropanol, Zucker, wie Aldosen und Ketosen in Form von Monosacchariden, Disacchariden und Oligosacchariden, insbesondere Glukose, Fruktose und Laktose, Aldehyde, wie Formaldehyd, Bor-Wasserstoff-Verbindungen, wie Borhydride, Borane, Metallboranate und Borankomplexe, wie Diboran, Natriumborhydrid und Aminoborane, insbesondere Trimethylaminoboran, Hydrazin und Alkylhydrazine, wie Methylhydrazin, Hydrogendithionite und Dithionite, insbesondere Natrium- und Kaliumhydrogendithionit, Natrium-, Kalium- und Zinkdithionite, Hydrogensulfide und Sulfide, insbesondere Natrium- und Kaliumhydrogensulfide, Natrium-Kalium- und Calciumsulfide, Hydroxylamin und Harnstoff, und Mischungen davon.

Für die Hydrierung eignen sich z. B. Katalysatoren, die auf einem Träger aus Kieselsäure Nickel und Kupfer neben anderen Metallen als aktive Bestandteile enthalten. Solche Katalysatoren werden z. B. in DE-A 26 28 987 beschrieben. Die aktive Masse dieser Katalysatoren enthält speziell 40 bis 80 Gew.-% Nickel, 10 bis 50 Gew.-% Kupfer und 2 bis 10 Gew.-% Mangan.

Die EP-A-0 434 062 beschreibt Hydrierungskatalysatoren, die durch Reduktion eines Precursors aus Oxiden des Kupfers, Aluminiums und wenigstens eines weiteren Metalls, ausgewählt unter Magnesium, Zink, Titan, Zirkon, Zinn, Nickel und Kobalt, erhältlich sind.

Geeignet sind auch die in der DE 102 18 849 beschriebenen Hydrierkatalysatoren, die 0,1 bis 10 Gew.-% Chrom, berechnet als Cr₂O₃, 0,1 bis 10 Gew.-% Calcium, berechnet als CaOₓ und 5 bis 20 Gew.-% Kupfer, berechnet als CuO, abgeschieden auf einem Siliciumdioxid-Trägermaterial und jeweils bezogen auf das Gesamtgewicht des calcinierten Katalysators, enthalten.

Aus der DE-A-40 21 230 sind Kupfer-Zirkonoxid-Katalysatoren bekannt, wobei das Verhältnis von Kupferatomen zu Zirkoniumatomen, ausgedrückt als Gewichtsverhältnis, 1 : 9 bis 9 : 1 beträgt.

Die DE-A-4 028 295 beschreibt Kupfer-Mangan-Hydrierkatalysatoren.

EP-A-552463 beschreibt in einer ersten Ausführungsform Hydrierkatalysatoren, wobei die oxidische Form im Wesentlichen der Zusammensetzung CuₐAl_{b}Zr_{c}Mn_{d}Oₓ entspricht, wobei die folgenden Beziehungen gelten: a > 0; b > 0; c ≥ 0; d > 0; a > b/2; b > a/4; a > c; a > d; und x die zur Wahrung der Elektroneutralität pro Formeleinheit erforderliche Anzahl von Sauerstoffionen bezeichnet. Nach einer weiteren Ausführungsform enthält der erfindungsgemäße Katalysator einen geringeren Anteil an Aluminiumoxid. Der Katalysator nach dieser Ausführungsform entspricht im Wesentlichen der Zusammensetzung CuₐAl_{b}Zr_{c}Mn_{d}Oₓ, wobei die folgenden Beziehungen gelten: a > 0; a/4 ≥ b ≥ a/40 ; c ≥ 0; d > 0; a > c; 0,95d ≥ a ≥ 0,5d und x die zur Wahrung der Elektroneutralität pro Formeleinheit erforderliche Anzahl von Sauerstoffionen bezeichnet.

Die WO 2006/005505 beschreibt Katalysatorformkörper, die sich für den Einsatz in dem erfindungsgemäßen Verfahren besonders eignen. Diese können durch ein Verfahren hergestellt werden, bei dem,
(i) ein oxidisches Material, umfassend Kupferoxid, Aluminiumoxid und mindestens eines der Oxide des Lanthans, Wolframs, Molybdäns, Titans oder Zirkoniums wobei die Oxide des Lanthans und/oder Wolframs bevorzugt sein sollen, bereitgestellt wird,
(ii) dem oxidischen Material pulverförmiges metallisches Kupfer, Kupferblättchen, pulverförmiger Zement oder deren Gemische oder ein Gemisch derselben mit Graphit zugegeben werden kann, und
(iii) das aus (ii) resultierende Gemisch zu einer Katalysatortablette oder einem Katalysatorextrudat mit einem Durchmesser d und/oder einer Höhe h < 6,0 mm, Katalysatorkugeln mit einem Durchmesser d < 6,0 mm oder Katalysator-Wabenkörper mit einem Zelldurchmesser rz < 6,0 mm verformt wird.

Von den Oxiden des Lanthans, Wolframs, Molybdäns, Titans oder Zirkoniums ist Lanthanoxid bevorzugt. Die Zusammensetzung des oxidischen Materials ist im Allgemeinen so beschaffen, dass der Anteil an Kupferoxid im Bereich von 40 bis 90 Gew.-%, der Anteil an Oxiden des Lanthans, Wolframs, Molybdäns, Titans oder Zirkoniums im Bereich von 0 bis 50 Gew.-% und der Anteil an Aluminiumoxid im Bereich bis zu 50 Gew.-%, jeweils bezogen auf das Gesamtgewicht der Summe der oben genannten oxidischen Bestandteile, liegt, wobei diese drei Oxide zusammen mindestens 80 Gew.-% des oxidischen Materials nach Calcinierung darstellen, wobei Zement nicht dem oxidischen Material in obigem Sinne zugerechnet wird.

In einer bevorzugten Ausführung umfasst das oxidische Material
(a) Kupferoxid mit einem Anteil im Bereich von 50 ≤ x ≤ 80 Gew.-%, bevorzugt 55 ≤ x ≤ 75 Gew.-%,
(b) Aluminumoxid mit einem Anteil im Bereich von 15 ≤ y ≤ 35 Gew.-%, bevorzugt 20 ≤ y ≤ 30 Gew.-%, und
(c) mindestens eines der Oxide des Lanthans, Wolframs, Molybdäns, Titans oder Zirkoniums, bevorzugt des Lanthans und/oder Wolframs, mit einem Anteil im Bereich von 2 ≤ z ≤ 20 Gew.-%, bevorzugt 3 ≤ z ≤ 15 Gew.-%,
jeweils bezogen auf das Gesamtgewicht des oxidischen Materials nach Calcinierung, wobei gilt: 80 ≤ x + y + z ≤ 100, besonders 95 ≤ x + y + z ≤ 100.

Bevorzugte Katalysatoren umfassen die folgenden Metalle in oxidischer Form, reduzierter Form (elementarer Form) oder einer Kombination davon. Metalle, die in mehr als einer Oxidationsstufe stabil sind, können vollständig in einer der Oxidationsstufen oder in verschiedenen Oxidationsstufen eingesetzt werden:
Cu
Cu, Ti
Cu, Zr
Cu, Mn
Cu, Al
Cu, Ni, Mn
Cu, Al, wenigstens ein weiteres Metall, ausgewählt unter La, W, Mo, Mn, Zn, Ti, Zr, Sn, Ni, Co
Cu, Zn, Zr
Cu, Cr, Ca
Cu, Cr, C
Cu, Al, Mn, gegebenenfalls Zr

Besonders bevorzugte Katalysatoren umfassen die folgenden Metalle:
Cu
Cu, Ti
Cu, Al
Cu, Al, La
Cu, Al, Zn
Cu, Zn, Zr
Cu, Al, Mn
Cu, Cr, C

Als inertes Trägermaterial für die erfindungsgemäßen Katalysatoren können praktisch alle Trägermaterialien des Stands der Technik, wie sie vorteilhaft bei der Herstellung von geträgerten Katalysatoren Verwendung finden, beispielsweise SiO₂ (Quarz), Porzellan, Magnesiumoxid, Zinndioxid, Siliciumcarbid, TiO₂ (Rutil, Anatas), Al₂O₃ (Tonerde), Aluminiumsilikat, Steatit (Magnesiumsilikat), Zirkoniumsilikat, Cersilikat oder Mischungen dieser Trägermaterialien, eingesetzt werden. Bevorzugte Trägermaterialien sind Aluminiumoxid und Siliciumdioxid. Als Siliciumdioxid-Trägermaterial können Siliciumdioxid-Materialien unterschiedlicher Herkunft und Herstellung, z. B. pyrogen erzeugte Kieselsäuren oder nasschemisch hergestellte Kieselsäuren, wie Kieselgele, Aerogele oder Fällungskieselsäuren, zur Katalysatorherstellung eingesetzt werden (zur Herstellung der verschiedenen SiO₂-Ausgangsmaterialien siehe: W. Büchner; R. Schliebs; G. Winter; K. H. Büchel: Industrielle Anorganische Chemie; 2. Aufl., S. 532 - 533, VCH Verlagsgesellschaft, Weinheim 1986).

Die Katalysatoren können als Formkörper eingesetzt werden, z. B. in Form von Kugeln, Ringen, Zylindern, Würfeln, Quadern oder anderen geometrischen Körpern. Ungeträgerte Katalysatoren können nach üblichen Verfahren geformt werden, z. B. durch Extrudieren, Tablettieren, etc. Die Form geträgerter Katalysatoren wird durch die Form des Trägers bestimmt. Alternativ dazu kann der Träger vor oder nach dem Aufbringen der katalytisch aktiven Komponente(n) einem Formungsverfahren unterzogen werden. Die Katalysatoren können z. B. in Form von gepressten Zylindern, Tabletten, Pastillen, Wagenrädern, Ringen, Sternen oder Strangpresslingen, wie Vollsträngen, polylobären Strängen, Hohlsträngen und Wabenkörpern oder anderen geometrischen Körpern, eingesetzt werden.

Die Katalysatorteilchen weisen im Allgemeinen einen Mittelwert des (größten) Durchmessers von 0,5 bis 20 mm, vorzugsweise 1 bis 10 mm, auf. Dazu zählen z. B. Katalysatoren in Form von Tabletten, z. B. mit einem Durchmesser von 1 bis 7 mm, vorzugsweise 2 bis 6 mm, und einer Höhe von 3 bis 5 mm, Ringen mit z. B. 4 bis 7 mm, vorzugsweise 5 bis 7 mm, Außendurchmesser, 2 bis 5 mm Höhe und 2 bis 3 mm Lochdurchmesser, oder Strängen unterschiedlicher Länge eines Durchmessers von z. B. 1,0 bis 5 mm. Derartige Formen können auf an sich bekannte Weise durch Tablettierung, Strangpressen oder Extrusion erhalten werden. Dazu können der Katalysatormasse übliche Hilfsmittel, z. B. Gleitmittel, wie Graphit, Polyethylenoxid, Cellulose oder Fettsäuren (wie Stearinsäure), und/oder Formhilfsmittel und Verstärkungsmittel, wie Fasern aus Glas, Asbest oder Siliciumcarbid, zugesetzt werden.

Eine spezielle Ausführungsform geträgerter Katalysatoren sind Schalenkatalysatoren. Bevorzugt eignen sich für das erfindungsgemäße Verfahren auch Schalenkatalysatoren. Schalenkatalysatoren umfassen eine schalenförmig auf einen Träger aufgebrachte katalytische Masse. Sie können in Form von Kugeln, Ringen, Zylindern, Würfeln, Quadern oder anderen geometrischen Körpern vorliegen. Unabhängig von der Art und Zusammensetzung des katalytisch aktiven Materials kann die Bereitstellung von Schalenkatalysatorteilchen prinzipiell erfolgen, indem man den Träger mit einem flüssigen Bindemittel und der katalytisch aktiven Masse in Kontakt bringt, dabei eine Schicht der Masse auf dem Träger aufbringt, und anschließend gegebenenfalls das Bindemittel teilweise entfernt. Dabei wird zur Bereitstellung der Katalysatorteilchen das katalytisch aktive Material bereits in seiner fertigen katalytisch aktiven Form, beispielsweise als calciniertes Mischoxid, aufgebracht. Geeignete Verfahren zur Herstellung von Schalenkatalysatoren sind z. B. in der DE-A-29 09 671 und in der EP-A-714 700 beschrieben. Nach dem letztgenannten Verfahren wird der Träger zunächst mit dem flüssigen Bindemittel befeuchtet, dann durch Inkontaktbringen mit trockener, feinteiliger, aktiver Katalysatormasse an der Oberfläche des befeuchteten Trägerkörpers eine Schicht aktiver Katalysatormasse angeheftet und anschließend gegebenenfalls das flüssige Bindemittel teilweise entfernt. In einer speziellen Ausführung werden die Schritte des Befeuchtens des Trägers, des Inkontaktbringens mit der Katalysatormasse und des Entfernens des flüssigen Bindemittels ein- oder mehrfach wiederholt, bis die gewünschte Schichtdicke des Schalenkatalysators erreicht ist.

Eine weitere spezielle Ausführungsform geträgerter Katalysatoren sind durch Tränkverfahren hergestellte Katalysatoren. Dazu können die katalytisch aktiven Katalysatorkomponenten oder Vorläuferverbindungen davon auf das Trägermaterial aufgetragen werden. Im Allgemeinen werden zum Tränken des Trägermaterials wässrige Salzlösungen der Komponenten, z. B. wässrige Lösungen von deren Halogeniden, Sulfaten, Nitraten, etc. aufgebracht. Die Kupferkomponente kann z. B. auch in Form einer wässrigen Lösung ihrer Aminkomplexsalze, beispielsweise als [Cu(NH₃)₄]SO₄- oder als [Cu(NH₃)₄](NO₃)₂-Lösung, gegebenenfalls in Gegenwart von Natriumcarbonat, auf das Trägermaterial aufgetragen werden. Selbstverständlich können auch andere Kupferaminkomplexe als die beispielhaft genannten mit gleichem Erfolg für die Katalysatorherstellung verwendet werden.

Die Imprägnierung des Trägermaterials mit den Vorläuferverbindungen der katalytisch aktiven Komponenten kann prinzipiell einstufig oder mehrstufig erfolgen. Die Imprägnierung kann in herkömmlichen Tränkvorrichtungen, z. B. Imprägniertrommeln, vorgenommen werden. Nach Trocknung und/oder Calcinierung erhält man dann den fertigen Katalysator. Die Trocknung der getränkten Katalysatorformkörper kann kontinuierlich oder chargenweise, z. B. in Band- oder Hordenöfen, erfolgen. Die Trocknung kann bei Atmosphärendruck oder vermindertem Druck erfolgen. Des Weiteren kann die Trocknung in einem Gasstrom, z. B. einem Luftstrom oder einen Stickstoffstrom, erfolgen. Je nach angewandtem Druck wird die Trocknung im Allgemeinen bei Temperaturen von 50 bis 200 °C, vorzugsweise 80 bis 150 °C durchgeführt. Die Calcinierung des gegebenenfalls zuvor getrockneten Katalysators erfolgt im Allgemeinen bei Temperaturen von 200 bis 800 °C, vorzugsweise 500 bis 700 °C. Die Calcinierung kann, wie die Trocknung, kontinuierlich oder chargenweise, z. B. in Band- oder Hordenöfen, durchgeführt werden. Die Calcinierung kann bei Atmosphärendruck oder vermindertem Druck und/oder in einem Gasstrom erfolgen, z. B. in einem Luft- oder einem Wasserstoffstrom. Eine Vorbehandlung mit Wasserstoff oder Wasserstoff enthaltenden Gasen im Allgemeinen unter Bedingungen, die den Hydrierbedingungen entsprechen, dient der Vorreduzierung/Aktivierung des Hydrierkatalysators. Der Katalysator kann aber auch in situ unter den bei der Hydrierung vorgegebenen Bedingungen, vorzugsweise unter Druck (z. B. bei einem Wasserstoffdruck von etwa 100 bis 325 bar), reduziert werden.

Der Hydrieraustrag besteht im Wesentlichen aus 1,2-Propandiol. Weitere Bestandteile sind u. a. Methanol, Ethanol, n-Propanol, Isopropanol, 1,3-Propandiol, Glycerin, Ethylenglykol und Wasser. Der Hydrieraustrag kann anschließend nach üblichen, dem Fachmann bekannten Verfahren aufgearbeitet werden. Dazu eignen sich beispielsweise thermische Verfahren, vorzugsweise destillative Verfahren, Adsorption, lonenaustausch, Membrantrennverfahren, Kristallisation, Extraktion oder eine Kombination von zwei oder mehreren dieser Verfahren. Bevorzugt wird der Hydrieraustrag durch Destillation aufgearbeitet. Dazu eignen sich übliche, dem Fachmann bekannte Destillationsverfahren. Geeignete Vorrichtungen für die destillative Aufarbeitung umfassen Destillationskolonnen, wie Bodenkolonnen, die mit Glocken, Siebplatten, Siebböden, Packungen, Einbauten, Ventilen, Seitenabzügen, etc. versehen sein können. Geeignet sind speziell Trennwandkolonnen, die mit Seitenabzügen, Rückführungen, etc. versehen sein können. Zur Destillation kann eine Kombination aus zwei oder mehr als zwei Destillationskolonnen eingesetzt werden. Geeignet sind weiterhin Verdampfer, wie Dünnschichtverdampfer, Fallfilmverdampfer, Sambay-Verdampfer, etc. und Kombinationen davon. Im Hydrieraustrag noch enthaltenes Glycerin kann, gegebenenfalls nach destillativer Abtrennung, in die Hydrierstufe zurückgeführt werden.

Die Erfindung wird anhand der folgenden, nicht einschränkenden Beispiele näher erläutert.

### Beispiele

Figur 1 zeigt die schematische Darstellung der zur Durchführung des Hydrierverfahrens nach Beispiel 1 eingesetzten zweistufigen Reaktorkaskade, wobei aus Gründen der Übersichtlichkeit auf die Wiedergabe solcher Details verzichtet ist, die für die Erläuterung der Erfindung nicht relevant sind.

### Beispiel 1

Als Reaktor (1) wird ein Schachtreaktor mit 540 I Katalysatorvolumen eingesetzt. Dieser enthält als Katalysator ein oxidisches Material eingebaut, das CuO_{0,6-0,85} (Al₂O₃)_{0,1-0,34} (La₂O₃)_{0,01-0},₂ umfasst und welches, bezogen auf das Gesamtgewicht des oxidischen Materials, bis zu 20 % Hilfsstoffe enthalten kann. Der Katalysator wird vor der Hydrierreaktion im Wasserstoffstrom reduziert. Der ohne Kreisstrom ausgebildete Nachreaktor (8) hat ein Katalysatorvolumen von 120 I und enthält den gleichen Katalysator wie der Hauptreaktor (1).

Als Zuführstrom (3) wird Reinglycerin mit Wasser in einem Massenverhältnis von Glycerin zu Wasser von 9 : 1 vermischt. Der Feedstrom (3) (ca. 110 kg/h) wird mit einer Pumpe (nicht abgebildet) auf Reaktionsdruck gebracht und zusammen mit dem Rücklaufstrom (7) dem Reaktor bei einer Temperatur von 175 °C zugefahren.

Die Reaktion zum 1,2-Propandiol wird im Reaktor (1) bei einem Druck von 200 bar durchgeführt. Dafür wird druckgeregelt Wasserstoff über die Leitung (2) dem Reaktor (1) zugeführt. Die Wasserstoffmenge, die in den Reaktor gegeben wird, ergibt sich aus dem Reaktionsverbrauch und der Abgasmenge (stöchiometrischer Wasserstoffüberschuss 10 mol-% bis 25 mol-%). Der Reaktor (1) wird im Gleichstrom von oben nach unten durchströmt. Der Umsatz im Hauptreaktor beträgt ca. 93 bis 94 %. Der zweiphasige Reaktoraustrag (4) wird im Abscheider (10) in eine gasreiche und eine Flüssigphase getrennt. Die Flüssigphase wird über die Pumpe (5) als Kreisstrom zunächst zur Kühlung dem Wärmetauscher (6) zugeführt. Die Temperatur des Rückführstroms wird im Wärmetauscher (6) so eingestellt, dass mit der Mischung des Feedstroms (3) eine Eintrittstemperatur von 175 °C am Kopf des Reaktors erreicht wird. Das Rückführverhältnis (Rückführstrom (7) zu Feedstrom (3)) beträgt ca. 13. Der Reaktor (1) wird durch den Kreisstrom und den zugeführten kalten glycerinhaltigen feed (3) ausreichend gekühlt. Der aus dem Abscheider (10) entnommene zweite Teilstrom wird in einem weiteren Kühler (nicht abgebildet) auf die Eintrittstemperatur des Nachreaktors von ca. 175 °C abgekühlt. Der abgekühlte, zweiphasige Strom wird dem Kopf des Nachreaktors (8) zugeführt. Im Reaktor (8) wird die Hydrierung bis zu einem Gesamtumsatz von ca. 99 % weitergeführt.

## Patentansprüche

1. Verfahren zur Herstellung von 1,2-Propandiol, bei dem man
a) einen glycerinhaltigen Strom bereitstellt, und
b) den glycerinhaltigen Strom einer kontinuierlichen Hydrierung in zwei hintereinander geschalteten Hydrierreaktoren in Gegenwart eines kupferhaltigen, heterogenen Katalysators unterzieht, wobei der Umsatz im ersten Reaktor wenigstens 80 Gew.-%, bezogen auf den Glyceringehalt, beträgt und wobei die kontinuierliche Hydrierung in beiden Hydrierreaktoren bei einem Druck im Bereich von 30 bis 300 bar erfolgt.

2. Verfahren nach einem der vorhergehenden Ansprüche, wobei die kontinuierlichen Hydrierung in beiden Hydrierreaktoren bei einem Druck im Bereich von 60 bis 250 bar, erfolgt.

3. Verfahren nach einem der vorhergehenden Ansprüche, wobei der Gesamtglycerinumsatz in Schritt b), bezogen auf das in dem glycerinhaltigen Strom enthaltene Glycerin, wenigstens 97 %, bevorzugt wenigstens 98 %, insbesondere wenigstens 99 %, beträgt.

4. Verfahren nach einem der vorhergehenden Ansprüche, wobei der erste Hydrierreaktor über einen in einem externen Kreislauf geführten Strom aus der Reaktionszone verfügt.

5. Verfahren nach einem der vorhergehenden Ansprüche, wobei die Umsetzung in dem zweiten Reaktor adiabatisch durchgeführt wird.

6. Verfahren nach einem der vorhergehenden Ansprüche, wobei die Einspeisung von Wasserstoff nur in den ersten Reaktor erfolgt.

7. Verfahren nach einem der vorhergehenden Ansprüche, bei dem man in Verfahrensschritt b)
b1) in den ersten Reaktor eines Reaktionssystems, das aus einem Reaktor mit einen in einem externen Kreislauf geführten Strom und einem nachgeschalteten im geraden Durchgang betrieben Reaktor besteht, einen glycerinhaltigen Zulauf und Wasserstoff einspeist und in Gegenwart eines kupferhaltigen, heterogenen Katalysators bis zu einem Teilumsatz umsetzt,
b2) aus dem ersten Reaktor einen Austrag entnimmt, der Wasserstoff, Glycerin und 1,2-Propandiol enthält,
b3) den Austrag in einen ersten flüssigen, an Wasserstoff abgereicherten Teilstrom und einen zweiten an Wasserstoff angereicherten Teilstrom auftrennt,
b4) den ersten Teilstrom nach Entzug eines Teils der enthaltenen Wärme in den ersten Reaktor zurückführt, und
b5) den zweiten Teilstrom in den zweiten Reaktor einspeist und in Gegenwart eines kupferhaltigen, heterogenen Katalysators weiter umsetzt.

8. Verfahren nach einem der vorhergehenden Ansprüche, bei dem man in Schritt a) einen bei der Herstellung von Alkylestern höherer Fettsäuren durch Umesterung von Fettsäuretriglyceriden anfallenden glycerinhaltigen Strom bereitstellt.

9. Verfahren nach einem der Ansprüche 1 bis 8, wobei der glycerinhaltige Strom einen Wassergehalt von höchstens 30 Gew.-%, bevorzugt von höchstens 20 Gew.-%, aufweist.

10. Verfahren nach einem der Ansprüche 1 bis 8, wobei der glycerinhaltige Strom einen Wassergehalt von höchstens 3 Gew.-% aufweist.

11. Verfahren nach einem der vorhergehenden Ansprüche, bei dem man in Schritt a) den glycerinhaltigen Strom einer Aufarbeitung durch wenigstens ein Aufarbeitungsverfahren unterzieht, das ausgewählt ist unter thermischer Aufarbeitung, Adsorption, Ionenaustausch, Membrantrennung, Kristallisation, Extraktion oder eine Kombination aus zwei oder mehreren dieser Verfahren.

12. Verfahren nach einem der vorhergehenden Ansprüche, bei dem man in Schritt a) den glycerinhaltigen Strom einer Destillation zur Verringerung des Wassergehalts und/oder zur Entfernung von Komponenten, welche die katalytische Hydrierung beeinträchtigen, unterzieht.

13. Verfahren nach einem der vorhergehenden Ansprüche, bei dem man in Schritt a) den glycerinhaltigen Strom zur Verringerung des Gehalts an schwefelhaltigen Verbindungen, speziell schwefelhaltigen aromatischen Verbindungen, einer katalytischen Entschwefelung, gegebenenfalls in Gegenwart von Wasserstoff, unterzieht.

14. Verfahren nach einem der vorhergehenden Ansprüche, bei dem man in Schritt a) den glycerinhaltigen Strom zur Entfernung von Komponenten, welche die katalytische Hydrierung beeinträchtigen, mit wenigstens einem Adsorptionsmittel in Kontakt bringt.

15. Verfahren nach einem der vorhergehenden Ansprüche, wobei die Bereitstellung des glycerinhaltigen Stroms in Schritt a) die folgenden Schritte umfasst:
a1) Bereitstellen eines biogenen fett- und/oder ölhaltigen Ausgangsgemisches,
a2) Umestern der in dem Ausgangsgemisch enthaltenen Fettsäuretriglyceride mit wenigstens einem C₁-C₉-Monoalkohol und gegebenenfalls Verestern der in dem Ausgangsgemisch enthaltenen freien Fettsäuren unter Bildung eines Veresterungsgemisches,
a3) Auftrennen des Veresterungsgemisches unter Erhalt wenigstens einer an C₁-C₉-Monoalkylestern angereicherten Fraktion und wenigstens einer an bei der Umesterung freigesetztem Glycerin angereicherten Fraktion,
a4) gegebenenfalls Aufreinigen der an Glycerin angereicherten Fraktion.

## Claims

1. A process for preparing 1,2-propanediol, in which
a) a glycerol-containing stream is provided, and
b) the glycerol-containing stream is subjected to a continuous hydrogenation in two hydrogenation reactors connected in series in the presence of a heterogeneous copper catalyst, the conversion in the first reactor being at least 80%, based on the glycerol content, and the continuous hydrogenation in both hydrogenation reactors being effected at a pressure in the range from 30 to 300 bar.

2. The process according to the preceding claim, wherein the continuous hydrogenation in both hydrogenation reactors is effected at a pressure in the range from 60 to 250 bar.

3. The process according to any of the preceding claims, wherein the total glycerol conversion in step b), based on the glycerol present in the glycerol-containing stream, is at least 97%, preferably at least 98%, especially at least 99%.

4. The process according to any of the preceding claims, wherein the first hydrogenation reactor has a stream from the reaction zone conducted in an external circulation system.

5. The process according to any of the preceding claims, wherein the reaction is performed adiabatically in the second reactor.

6. The process according to any of the preceding claims, wherein hydrogen is fed only into the first reactor.

7. The process according to any of the preceding claims, in which, in process step b)
b1) a glycerol-containing feed and hydrogen are fed into the first reactor of a reaction system which consists of one reactor with a stream conducted in an external circulation system and a downstream reactor operated in straight pass, and converted up to a partial conversion in the presence of a heterogeneous copper catalyst,
b2) a discharge which comprises hydrogen, glycerol and 1,2-propanediol is withdrawn from the first reactor,
b3) the discharge is separated into a first liquid hydrogen-depleted substream and a second hydrogen-enriched substream,
b4) the first substream, after withdrawal of some of the heat contained, is recycled into the first reactor, and
b5) the second substream is fed into the second reactor and converted further in the presence of a heterogeneous copper catalyst.

8. The process according to any of the preceding claims, in which a glycerol-containing stream which is obtained in the preparation of alkyl esters of higher fatty acids by transesterification of fatty acid triglycerides is provided in step a).

9. The process according to any of claims 1 to 8, wherein the glycerol-containing stream has a water content of at most 30% by weight, preferably of at most 20% by weight.

10. The process according to any of claims 1 to 8, wherein the glycerol-containing stream has a water content of at most 3% by weight.

11. The process according to any of the preceding claims, in which the glycerol-containing stream is subjected in step a) to a workup by at least one workup process which is selected from thermal workup, adsorption, ion exchange, membrane separation, crystallization, extraction or a combination of two or more of these processes.

12. The process according to any of the preceding claims, in which the glycerol-containing stream is subjected in step a) to a distillation to reduce the water content and/or to remove components which impair the catalytic hydrogenation.

13. The process according to any of the preceding claims, in which the glycerol-containing stream is subjected in step a) to a catalytic desulfurization, optionally in the presence of hydrogen, to reduce the content of sulfur compounds, especially aromatic sulfur compounds.

14. The process according to any of the preceding claims, in which the glycerol-containing stream is contacted in step a) with at least one adsorbent to remove components which impair the catalytic hydrogenation.

15. The process according to any of the preceding claims, wherein the provision of the glycerol-containing stream in step a) comprises the following steps:
a1) providing a biogenic fat- and/or oil-containing starting mixture,
a2) transesterifying the fatty acid triglycerides present in the starting mixture with at least one C₁-C₉ monoalcohol and optionally esterifying the free fatty acids present in the starting mixture to form an esterification mixture,
a3) separating the esterification mixture to obtain at least one fraction enriched in C₁-C₉ monoalkyl esters and at least one fraction enriched in glycerol released in the transesterification,
a4) optionally, purifying the glycerol-enriched fraction.

## Revendications

1. Procédé pour la préparation de 1,2-propanediol, dans lequel
a) on prépare un flux contenant du glycérol et
b) on soumet le flux contenant du glycérol à une hydrogénation continue dans deux réacteurs d'hydrogénation disposés l'un derrière l'autre en présence d'un catalyseur hétérogène, contenant du cuivre, la conversion dans le premier réacteur étant d'au moins 80% en poids par rapport à la teneur en glycérol et l'hydrogénation continue dans les deux réacteurs d'hydrogénation ayant lieu à une pression dans la plage de 30 à 300 bars.

2. Procédé selon la revendication précédente, l'hydrogénation continue dans les deux réacteurs d'hydrogénation ayant lieu à une pression dans la plage de 60 à 250 bars.

3. Procédé selon l'une quelconque des revendications précédentes, la conversion totale du glycérol dans l'étape b), par rapport au glycérol contenu dans le flux contenant du glycérol, étant d'au moins 97%, de préférence d'au moins 98%, en particulier d'au moins 99%.

4. Procédé selon l'une quelconque des revendications précédentes, le premier réacteur d'hydrogénation disposant d'un flux guidé dans un circuit externe, provenant de la zone de réaction.

5. Procédé selon l'une quelconque des revendications précédentes, la transformation dans le deuxième réacteur étant réalisée de manière adiabatique.

6. Procédé selon l'une quelconque des revendications précédentes, l'injection d'hydrogène n'étant réalisée que dans le premier réacteur.

7. Procédé selon l'une quelconque des revendications précédentes, dans lequel, dans l'étape b),
b1) une alimentation, contenant du glycérol, et de l'hydrogène étant injectés dans le premier réacteur d'un système de réaction, qui est constitué par un réacteur présentant un flux guidé dans un circuit externe et un réacteur disposé en aval, exploité en passage droit, et transformés en présence d'un catalyseur hétérogène, contenant du cuivre, jusqu'à une conversion partielle,
b2) un produit est évacué du premier réacteur, qui contient de l'hydrogène, du glycérol et du 1,2-propanediol,
b3) le produit est séparé en un premier flux partiel liquide, appauvri en hydrogène, et un deuxième flux partiel, enrichi en hydrogène,
b4) le premier flux partiel est recyclé, après soutirage d'une partie de la chaleur contenue, dans le premier réacteur et
b5) le deuxième flux partiel est injecté dans le deuxième réacteur et transformé davantage en présence d'un catalyseur hétérogène, contenant du cuivre.

8. Procédé selon l'une quelconque des revendications précédentes, dans lequel, dans l'étape a), on met à disposition un flux contenant du glycérol produit lors de la préparation d'esters alkyliques d'acides gras supérieurs par transestérification de triglycérides d'acide gras.

9. Procédé selon l'une quelconque des revendications 1 à 8, le flux contenant du glycérol présentant une teneur en eau d'au plus 30% en poids, de préférence d'au plus 20% en poids.

10. Procédé selon l'une quelconque des revendications 1 à 8, le flux contenant du glycérol présentant une teneur en eau d'au plus 3% en poids.

11. Procédé selon l'une quelconque des revendications précédentes, dans lequel, dans l'étape a), on soumet le flux contenant du glycérol à un traitement par au moins un procédé de traitement qui est choisi parmi un traitement thermique, une adsorption, un échange d'ions, une séparation sur membrane, une cristallisation, une extraction ou une combinaison de deux de ces procédés ou plus.

12. Procédé selon l'une quelconque des revendications précédentes, dans lequel on soumet, dans l'étape a), le flux contenant du glycérol à une distillation pour diminuer la teneur en eau et/ou pour éliminer des composants qui compromettent l'hydrogénation catalytique.

13. Procédé selon l'une quelconque des revendications précédentes, dans lequel, dans l'étape a), on soumet le flux contenant du glycérol à une désulfuration catalytique, le cas échéant en présence d'hydrogène, pour diminuer la teneur en composés soufrés, en particulier en composés soufrés aromatiques.

14. Procédé selon l'une quelconque des revendications précédentes, dans lequel, dans l'étape a), on met en contact le flux contenant du glycérol avec au moins un adsorbant pour éliminer des composants qui compromettent l'hydrogénation catalytique.

15. Procédé selon l'une quelconque des revendications précédentes, dans lequel la préparation du flux contenant du glycérol dans l'étape a) comprend les étapes suivantes :
a1) mise à disposition d'un mélange de départ biogène contenant des graisses et/ou des huiles,
a2) transestérification des triglycérides d'acides gras contenus dans le mélange de départ avec au moins un monoalcool en C₁-C₉ et, le cas échéant, estérification des acides gras libres contenus dans le mélange de départ avec formation d'un mélange d'estérification,
a3) séparation du mélange d'estérification avec obtention d'au moins une fraction enrichie en esters monoalkyliques en C₁-C₉ et d'au moins une fraction enrichie en glycérol libéré lors de la transestérification,
a4) le cas échéant purification de la fraction enrichie en glycérol.
